# EUROPEAN PATENT APPLICATION

(11) **EP 4 407 623 A1**
(43) Date of publication of application: **31.07.2024**
(21) Application number: 23214621.7
(22) Date of filing: 06.12.2023
(51) Int. Cl.: G16B 20/30, G16B 50/00, G16B 20/00, G16B 30/10

(54) **METHODS AND SYSTEMS FOR HANDLING AUTOIMMUNE DISORDERS**

(30) Priority: 08.12.2022 IN 202221070990
(71) Applicant: Tata Consultancy Services Limited, Maharashtra (IN)
(72) Inventor: ANAND, SWADHA, 411013 Pune, Maharashtra (IN); BHATT, VINEET, 411013 Pune, Maharashtra (IN); MANDE, SHARMILA SHEKHAR, 411013 Pune, Maharashtra (IN)
(74) Representative: Goddar, Heinz J.

(57) **Abstract**

This disclosure relates generally to methods and systems for handling autoimmune disorders. Effective handling techniques for treating the autoimmune disorders are limited. The present disclosure herein solves the problem of treating and handling the autoimmune disorders effectively by identifying the microbial epitopes present in the sample of the subject and by mapping the identified epitopes through the epitope knowledgebase. In the present disclosure, a biological sample is collected from a subject. Next, one or more DNA sequences are extracted, and microbial taxa and one or more pathogenic microbes are identified. Further, one or more microbial epitopes from the biological sample are identified. the autoimmune disorders of the subject are then assessed, based on at least one of (i) the one or more pathogenic microbes and (ii) the one or more microbial epitopes, using the epitope knowledgebase, to generate an artificial sequence construct, using one or more mimic epitopes.

## Description

### CROSS-REFERENCE TO RELATED APPLICATIONS AND PRIORITY

The present application claims priority to Indian application no. 202221070990, filed on December 08, 2022.

### TECHNICAL FIELD

The disclosure herein generally relates to the field of autoimmune diseases, and, more particularly, to methods and systems for handling autoimmune disorders or diseases.

### BACKGROUND

An autoimmune disorder or disease is a condition arising from an abnormal immune response to a functioning body part. There are several types of autoimmune diseases that have been identified based on the body part involved. Some of the autoimmune disorders include but are not limited to primary biliary cholangitis (PBC) and atopic dermatitis (AD). Hence autoimmune disorders need to be handled using suitable therapeutic procedures. Conventional strategies include the use of synthetic and biological drugs for handling autoimmune disorders, these drugs are broad spectrum and non-disease specific with multiple side effects. Prolonged use of the conventional strategies can potentially increase the risk of developing deadly infections and cancers.

Further, the synthetic and biological drugs that are used to treat various autoimmune disorders inhibit various components of immune system such as co-stimulatory molecules and cytokines or intervene in immunoregulatory pathways in order to limit the immunogenic response in disease conditions. For example, Janus kinase (JAK) and Tyrosine Kinase 2 (TYK2) inhibitors are the primary drugs used for the treatment by blocking JAK-STAT pathway which control signaling of various interleukins and cytokines crucial for immune regulation. Hence, this approach is non-selective leading to secondary infections from bacteria, fungi, and viruses. Few other handling techniques for treating autoimmune disorders are under trial and the efficacy and safety of such techniques are still unclear. Hence the effective handling techniques for treating the autoimmune disorders are limited and there is room for improvement.

### SUMMARY

Embodiments of the present disclosure present technological improvements as solutions to one or more of the above-mentioned technical problems recognized by the inventors in conventional systems.

In one aspect, a method for handling autoimmune disorders is provided. The method comprising: collecting a biological sample from a subject for whom one or more autoimmune disorders to be handled; extracting one or more DNA sequences, from the biological sample, using one or more DNA sequence extraction techniques; identifying microbial taxa from the one or more DNA sequences of the biological sample, using one or more microbial taxa extraction techniques, wherein the microbial taxa is composed of one or more microbes predominantly present in the biological sample; identifying one or more pathogenic microbes, by mapping pathogenic microbial taxa obtained from the microbial taxa to the pathogenic microbial taxa present in a disease microbe map (DM map) of an epitope knowledgebase; identifying one or more microbial peptides from the biological sample, using one or more microbial peptides identification techniques; identifying one or more microbial epitopes, from the one or more microbial peptides present in the biological sample, using the epitope knowledgebase; assessing one or more autoimmune disorders of the subject, based on at least one of (i) the one or more pathogenic microbes and (ii) the one or more microbial epitopes, using the epitope knowledgebase; generating an artificial sequence construct, using one or more mimic epitopes identified from the epitope knowledgebase, wherein the artificial sequence construct comprises of one or more refined molecular mimic epitopes associated to the one or more autoimmune disorders present in the epitope knowledgebase linked by (i) one or more peptide linkers, (ii) one or more adjuvants, and (iii) one or more toll-like receptor (TLR) ligands, to enhance an immunogenicity; and assessing an efficacy of the artificial sequence construct generated to the subject.

In another aspect, a system for handling autoimmune disorders is provided. The system comprising: one or more hardware processors; a memory; input/output (I/O) interfaces; a sample collection module; a DNA extraction and sequencing module; a peptide identification module; an epitope identification module; an epitope knowledgebase; a disease assessment module; a sequence construct generation module; an efficacy assessment module; and wherein the one or more hardware processors are configured by the instructions to perform one or more of: collecting a biological sample from a subject for whom one or more autoimmune disorders to be handled, through the sample collection module; extracting one or more DNA sequences from the biological sample, using one or more DNA sequence extraction techniques, through the DNA extraction and sequencing module; identifying microbial taxa from the one or more DNA sequences of the biological sample, using one or more microbial taxa extraction techniques, wherein the microbial taxa is composed of one or more microbes predominantly present in the biological sample, through the DNA extraction and sequencing module; identifying one or more pathogenic microbes, by mapping pathogenic microbial taxa obtained from the microbial taxa to the pathogenic microbial taxa present in a disease microbe map (DM map) of the epitope knowledgebase; identifying one or more microbial peptides from the biological sample, using one or more microbial peptides identification techniques, through the peptide identification module; identifying, one or more microbial epitopes, from the one or more microbial peptides present in the biological sample, using the epitope knowledgebase, through the epitope identification module; assessing the one or more autoimmune disorders of the subject, based on at least one of (i) the one or more pathogenic microbes and (ii) the one or more microbial epitopes, via one or more hardware processors, using the epitope knowledgebase, through the disease assessment module; generating an artificial sequence construct, using one or more mimic epitopes identified from the epitope knowledgebase through the sequence construct generation module, wherein the artificial sequence construct comprises of one or more refined molecular mimic epitopes associated to the one or more autoimmune disorders present in the epitope knowledgebase, linked by (i) one or more peptide linkers, (ii) one or more adjuvants, and (iii) one or more toll-like receptor (TLR) ligands, to enhance an immunogenicity; and assess an efficacy of the artificial sequence construct generated to the subject.

In yet another aspect, there are provided one or more non-transitory machine-readable information storage mediums comprising one or more instructions which when executed by one or more hardware processors cause: collecting a biological sample from a subject for whom one or more autoimmune disorders to be handled, through the sample collection module; extracting one or more DNA sequences from the biological sample, using one or more DNA sequence extraction techniques, through the DNA extraction and sequencing module; identifying microbial taxa from the one or more DNA sequences of the biological sample, using one or more microbial taxa extraction techniques, wherein the microbial taxa is composed of one or more microbes predominantly present in the biological sample, through the DNA extraction and sequencing module; identifying one or more pathogenic microbes, by mapping pathogenic microbial taxa obtained from the microbial taxa to the pathogenic microbial taxa present in a disease microbe map (DM map) of the epitope knowledgebase; identifying one or more microbial peptides from the biological sample, using one or more microbial peptides identification techniques, through the peptide identification module; identifying one or more microbial epitopes, from the one or more microbial peptides present in the biological sample, using the epitope knowledgebase, through the epitope identification module; assessing the one or more autoimmune disorders of the subject, based on at least one of (i) the one or more pathogenic microbes and (ii) the one or more microbial epitopes, via one or more hardware processors, using the epitope knowledgebase, through the disease assessment module; generating an artificial sequence construct, using one or more mimic epitopes identified from the epitope knowledgebase through the sequence construct generation module, wherein the artificial sequence construct comprises of one or more refined molecular mimic epitopes associated to the one or more autoimmune disorders present in the epitope knowledgebase, linked by (i) one or more peptide linkers, (ii) one or more adjuvants, and (iii) one or more toll-like receptor (TLR) ligands, to enhance an immunogenicity; and assessing an efficacy of the artificial sequence construct generated to the subject.

In an embodiment, the one or more refined molecular mimic epitopes associated to the one or more autoimmune disorders are utilized to generate one or more monoclonal antibodies for administering the subject to impede a disease progression.

In an embodiment, the one or more peptide linkers are selected from a group consisting of: GGGS, AAY, KK, GPGPG, and HEYGAEALERAG, and wherein B cell epitopes and HTL epitopes (MHC II epitopes) can be linked by KK and GPGPG peptide linker; the one or more adjuvants are selected from a group consisting of: particulate emulsions, microparticles, iscoms cochleates, and liposomes, and wherein the one or more adjuvants are fused using a EAAAK linker; and the one or more toll-like receptor (TLR) ligands are selected from a group consisting of: MPL (TLR2 and TLR4 ligands), CpG ODN (TLR9 ligand), CTB-CpG, Flagellin (TLR5 ligand).

In an embodiment, the one or more pathogenic microbes comprising the epitope identified in a disease condition is eliminated using antimicrobials, wherein the antimicrobials comprise a microbial modulation in the form of probiotics, prebiotics or the antimicrobials that target the one or more pathogenic microbes in the biological sample in order to control disease progression.

In an embodiment, the artificial sequence construct comprises a combination of one or more epitope sequences from a sequence identifier (ID) D2_1 to D2_16 listed in Table 4, the one or more peptide linkers, the one or more adjuvants or the one or more toll-like receptor (TLR) ligands, for an autoimmune disorder being a primary biliary cholangitis (PBC).

In an embodiment, the artificial sequence construct comprises a combination of one or more epitope sequences from a sequence identifier (ID) D1_1 to D1_35 listed in Table 5, the one or more peptide linkers, the one or more adjuvants or the one or more toll-like receptor (TLR) ligands, for an autoimmune disorder being an Atopic Dermatitis (AD).

In an embodiment, the epitope knowledgebase comprises a DM_map, a X_RMME_map, and a X_RMME_detail_map, wherein the DM_map comprises mapping of the one or more autoimmune disorders to a set of microbes, the X_RMME_map comprises mapping of one or more refined molecular mimic epitopes to each of the one or more autoimmune disorders, and the X_RMME_detail_map comprises a detailed information of each of the one or more refined molecular mimic epitopes present in the X_RMME_map.

In an embodiment, the one or more refined molecular mimic epitopes of an autoimmune disorder being a primary biliary cholangitis (PBC), are listed in Table 4 in the form of epitope sequences from a sequence identifier (ID) D2_1 to D2_16. In an embodiment, the one or more refined molecular mimic epitopes of an autoimmune disorder being an Atopic Dermatitis (AD), are listed in Table 5 in the form of epitope sequences from a sequence identifier (ID) D1_1 to D1_35.

In an embodiment, the epitope knowledgebase is created by: identifying a plurality of disease specific proteomes pertaining to the one or more autoimmune disorders, using one or more data mining techniques; predicting one or more disease specific epitopes for each of the one or more autoimmune disorders, from the plurality of disease specific proteomes, based on a binding capability; identifying one or more potential molecular mimic epitopes, for each of the one or more autoimmune disorders, from the one or more disease specific epitopes, that show a sequence similarity with self-peptides and results in cross-activation of autoreactive T; and refining the one or more potential molecular mimic epitopes, to obtain one or more refined molecular mimic epitopes for each of the one or more autoimmune disorders, using one or more of (i) a structural superimposition and analysis, (ii) a cellular localization prediction, (iii) a proteosome processing, and (iv) an immunogenicity prediction.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary system for handling autoimmune disorders, according to some embodiments of the present disclosure.
FIG. 2A and 2B are flowcharts illustrating a method for handling autoimmune disorders, according to some embodiments of the present disclosure.
FIG. 3A through 3C are flowcharts illustrating a process for identifying mimic epitopes that are used for creating the epitope knowledgebase, according to some embodiments of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following embodiments described herein.

Mimic epitopes found in microbial proteins (referred as microbial peptides) show significant sequence and structural similarity to human peptides and are capable of eliciting immune response in various autoimmune conditions aggravating the disease. The present disclosure herein makes use of the identified peptides present in the microbial proteins and synthetically constructed epitopes as potential therapeutic and/or diagnostic agents for handling the autoimmune disorders such as primary biliary cholangitis (PBC) and atopic derma (AD).

The embodiments of present disclosure herein solve the problem of treating and handling the autoimmune disorders effectively by identifying the microbial epitopes present in a sample of a subject and by mapping the identified epitopes through the epitope knowledgebase.

In the context of the present disclosure, the definitions of some of the terms, keywords, or phrases are explained below:

The terms/expressions 'autoimmunity' and 'autoimmune' means a medical condition arising where the body's immune system generates an immune response against self-peptides when triggered either by molecular mimics during bacterial infection or any other mechanism. The terms `autoimmunity or autoimmune disorder' or the `autoimmunity condition' or `autoimmune disease' mean that the subjects having a medical condition arising due to autoimmunity. The term 'efficacy' is defined as the outcome of the normal treatment process utilized to handle the autoimmune condition. The term `epitopes' means short peptide fragments within a protein which have maximum capability to elicit an immune response by activation of immune cells. The terms `mimics' or `mimicry epitopes' mean the epitopes/ or short peptides present in the microbial proteins which show significant sequence and structural similarly to human peptides and are capable of eliciting an immune response in various autoimmune conditions aggravating the disease.

The term/expression `peptides' mean relatively the shorter sequences derived from the proteins. The term `microbial agents' are either the peptides, epitopes, RNA, DNA fragments or proteins derived from the microbes or the microbes itself. The term `microbe set' is the unique composition of the microbes at strain level defined in a specific disease state which are responsible for disease trigger or progression. The term `human proteome' is the set of entire protein sequences that are present and expressed in the human body. The term `human proteins' is the set of protein sequences from the entire pool of human proteome which are strongly associated with an autoimmune condition.

The term `microbial proteome' is the collective proteomes from all the microbes taken from the microbe set which is specific for an autoimmune condition. The term 'association' is the cause-and-effect relation of microbe to a particular autoimmune condition based on literature evidence. The term `epitope pool' means the epitopes identified for a particular autoimmune condition. The term `exogenous epitope pool (ExEP)' means the predicted epitopes which are derived from the proteins of microbial origin taken from microbial proteomes. The term `endogenous epitope pool (EnEP)' is the epitopes predicted from human proteome pool i.e., human proteins specific for a particular autoimmune disorder. The term 'superimposition' means the structural alignment of the mimic along with the protein sequences for which the mimic is identified in microbe and human. The terms 'subject', 'person', 'individual' means the living human being.

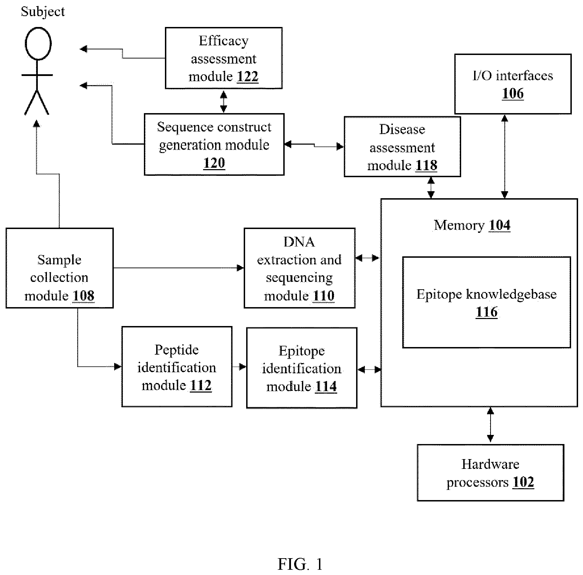

Referring now to the drawings, and more particularly to FIG. 1 through 3C, where similar reference characters denote corresponding features consistently throughout the figures, there are shown preferred embodiments and these embodiments are described in the context of the following exemplary system and/or method.

FIG. 1 illustrates an exemplary system 100 for handling autoimmune disorders, according to some embodiments of the present disclosure. In an embodiment, the system 100 includes or is otherwise in communication with one or more hardware processors 102, one or more data storage devices or memory 104 operatively coupled to the one or more hardware processors 102, communication interface device(s) or input/output (I/O) interface(s) 106, a sample collection module 108, a deoxyribonucleic acid (DNA) extraction and sequencing module 110, a peptide identification module 112, an epitope identification module 114, an epitope knowledgebase 116, a disease assessment module 118, a sequence construct generation module 120, and an efficacy assessment module 122. In an embodiment, the one or more hardware processors 102, the memory 104, and the I/O interface(s) 106 may be coupled to a system bus (not shown in FIG. 1) or a similar mechanism.

The I/O interface(s) 106 may include a variety of software and hardware interfaces, for example, a web interface, a graphical user interface, and the like. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface(s) 106 may include one or more ports for connecting a number of devices to one another or to another server.

The one or more hardware processors 102 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 102 are configured to fetch and execute computer-readable instructions stored in the memory 104. In the context of the present disclosure, the expressions 'processors' and `hardware processors' may be used interchangeably. In an embodiment, the system 100 can be implemented in a variety of computing systems, such as laptop computers, portable computers, notebooks, hand-held devices, workstations, mainframe computers, servers, a network cloud and the like.

The memory 104 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 102 includes a plurality of modules and a repository (not shown in FIG. 1) for storing data processed, received, and generated by one or more of the plurality of modules. The plurality of modules may include routines, programs, objects, components, data structures, and so on, which perform particular tasks or implement particular abstract data types.

The plurality of modules may include programs or computer-readable instructions or coded instructions that supplement applications or functions performed by the system 100. The plurality of modules may also be used as, signal processor(s), state machine(s), logic circuitries, and/or any other device or component that manipulates signals based on operational instructions. Further, the plurality of modules can be used by hardware, by computer-readable instructions executed by the one or more hardware processors 102, or by a combination thereof. In an embodiment, the plurality of modules can include various sub-modules (not shown in FIG. 1). Further, the memory 104 may include information pertaining to input(s)/output(s) of each step performed by the processor(s) 102 of the system 100 and methods of the present disclosure.

The repository may include a database or a data engine. Further, the repository amongst other things, may serve as a database or includes a plurality of databases for storing the data that is processed, received, or generated as a result of the execution of the plurality of modules. The repository may be internal or external to the system 100, where the repository may be stored within an external database (not shown in FIG. 1) communicatively coupled to the system 100. The data contained within such external database may be periodically updated. For example, data may be added into the external database and/or existing data may be modified and/or non-useful data may be deleted from the external database. In one example, the data may be stored in an external system, such as a Lightweight Directory Access Protocol (LDAP) directory and a Relational Database Management System (RDBMS). In another embodiment, the data stored in the repository may be distributed between the system 100 and the external database.

The sample collection module 108 is used to collect a biological sample from a subject to whom the autoimmune disorders to be handled. The DNA extraction and sequencing module 110 is used to isolate the DNA/ RNA from the biological sample and sequence the isolated DNA/ RNA to obtain DNA sequences. In an embodiment, the DNA extraction and sequencing module 110 may employ conventional standard laboratory procedures for isolating the DNA/ RNA and obtaining the DNA/ RNA sequences. The peptide identification module 112 is used to identify the peptides (microbial peptides) from the biological sample of the subject.

The epitope identification module 114 is used to identify the microbial epitopes from the microbial peptides present in the biological sample. The epitope knowledgebase 116 includes autoimmune disease specific epitope information. In an embodiment, the epitope knowledgebase 116 is present in the memory 104. The disease assessment module 118 includes various techniques in the art to utilize the identified disorder specific microbial agents captured in the epitope knowledgebase 116 which can be microbial mimic epitopes or microbes and detects the presence of these microbial agents in the biological sample of the subject for the presence or absence of the autoimmune disorders.

The sequence construct generation module 120 is used to generate an artificial sequence construct comprising one or more refined molecular mimic epitopes associated to the autoimmune disorders present in the epitope knowledgebase 116. The sequence construct generation module 120 prepares and administers the mentioned disease specific epitope mimic in one or more combinations as fusion constructs to design vaccines for tolerization of immune system using various administration methods to ameliorate the inflammation or disease condition. Also, the synthetically designed epitopes can be utilized to raise monoclonal antibodies by standard procedures to combat the disease. The one or more epitope sequences can be administered to the individual. The efficacy assessment module 122 assess the efficacy of the mentioned epitopes which are administered orally or topically or by any other administration process using the artificial sequence construct generated for the subject.

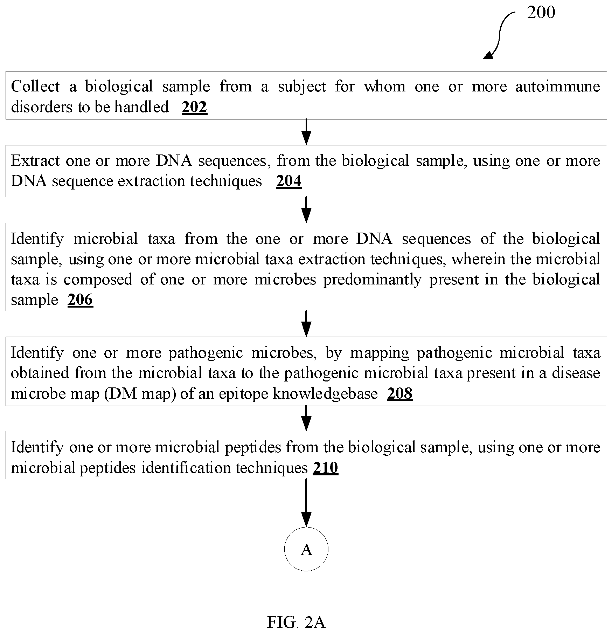

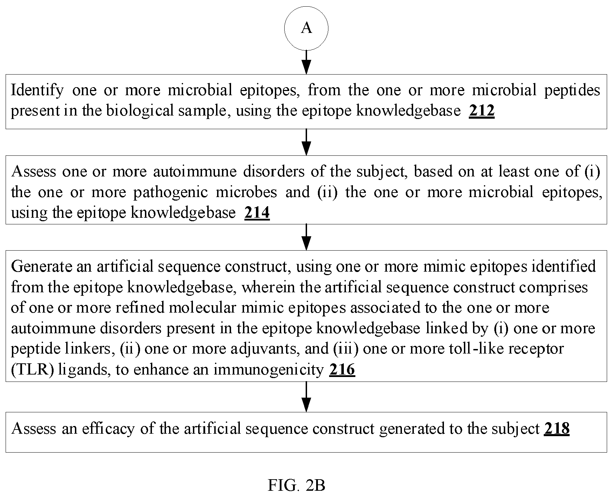

Referring to FIG. 2A and 2B, components and functionalities of the system 100 are described in accordance with an example embodiment of the present disclosure. FIG. 2A and 2B are flowcharts illustrating a method 200 for handling autoimmune disorders, according to some embodiments of the present disclosure. Although steps of the method 200 including process steps, method steps, techniques or the like may be described in a sequential order, such processes, methods and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

At step 202 of the method 200, the biological sample is collected from the subject to whom the autoimmune disorders to be handled, through the sample collection module 108. The biological sample is collected from the infected site which can be from the skin, gut or the oral mucosa or any other infected area from where the biological sample can be extracted using the standard laboratory procedures. The sample collection process largely depends on the site of infection, the disease and its pathophysiology. In autoimmune disorders affecting the skin, the biological sample is collected from the infected skin surface using skin swabs. A skin scrapping technique can also be used for sample collection from the infected area. In a few cases tissue biopsies can also be performed to collect the biological samples. In many autoimmune disorders microbiome dysbiosis occurs at more than one site (skin and the gut or the oral and gut) in combination in such scenarios the sample collection is done from respective sites using standard protocols and analyzed.

In one embodiment, the autoimmune disorders affecting skin may be but not be limited to one or more of PBC, AD, Psoriasis, etc. In another embodiment, the biological sample is obtained from the gut through fecal specimens, and the needle biopsy at the site of infection in the liver. The sample collection from the saliva and the mucosal linings can be performed using sterile swab (preferably cotton swabs) also a sterile syringe for the sample collection from the pus and aspirations of fluids.

In the case of infection present in the internal organ such as liver, lung, gut, joints etc. the sample collection technique depends on the organ from where the biological sample need to be collected. In one embodiment, the tissue biopsies can be performed at various sites while the sterile syringes can be used to obtain the fluid in the infected area to obtain sample. In case of lung infection endobronchial biopsies, sputum samples, oropharyngeal (OP) and nasopharyngeal (NP) swabs can be applied based on the site of infection. The biological samples can also be obtained using one or more of methods like the endoscopy biopsies or faecal specimens.

In liver related autoimmune disorders, the biological sample can be collected from liver biopsies using standard protocols, duodenal aspirate and biopsies which obtains the sample from descending and horizontal segments of the duodenum being anatomically close to liver and biliary tree. Needle biopsies can also be performed to extract infected fluid from the site. In one embodiment, the liver related autoimmune disorders might include one or more of disorders and ailments such as primary biliary cholangitis, autoimmune hepatitis (AIH), autoimmune sclerosing cholangitis, or any other liver related disorders are in the scope of the present disclosure. Similarly, the sample can be collected from other organs using conventional standard clinical procedures. In case of brain inflammations, the biological sample can be collected via CT (Computed Tomography)-guided aspiration, a type of needle biopsy and other biopsy procedures.

In another embodiment, the sera samples (biological samples) in the blood are collected to extract and identify the autoantigens, microbial peptides/epitopes and the autoantibodies involved in the disorder. The conventional standard techniques and protocols are used to obtain blood and sera samples. The microbial peptides present in the sera in either free form or in bound state with antibody can be detected along with other microbial agents implicated in the disease state. Acute serum can be collected from the individual for full metabolite and proteome profiling to detect range of microbial agents and the whole serum/blood sample can be submitted for bacterial culturing in order to detect microbes present in the infected sera. The urine samples can be collected for the urinary tract inflammations using laboratory procedures.

Further, at step 204 of the method 200, one or more DNA sequences are extracted from the biological sample of the subject collected at step 202 of the method 200, through the DNA extraction and sequencing module 110. The the DNA extraction and sequencing module 110 includes one or more DNA sequence extraction techniques available in the art for the DNA extraction and sequencing.

Further, at step 206 of the method 200, microbial taxa are identified from the one or more DNA sequences extracted at step 204 of the method 200, from the biological sample. In an embodiment, the DNA extraction and sequencing module 110 includes one or more microbial taxa extraction techniques available in the art for identifying the microbial taxa of the biological sample. The microbial taxa include one or more microbes predominantly present in the biological sample.

Further, at step 208 of the method 200, one or more pathogenic microbes are identified using the microbial taxa identified at step 206 of the method 200. In an embodiment, the epitope knowledgebase 116 is employed to identify the one or more pathogenic microbes from the microbial taxa. In an embodiment, the epitope knowledgebase 116 includes a disease microbe map (DM _map), a X_refined molecular mimic epitopes (RMME)_map, and a X_RMME_detail_map. The DM_map comprises mapping of the one or more autoimmune disorders to a set of microbes. The X_ RMME_map comprises mapping of one or more refined molecular mimic epitopes to each of the one or more autoimmune disorders. The X_RMME_detail_map comprises a detailed information of each of the one or more refined molecular mimic epitopes present in the X_RMME_map. More details about the epitope knowledgebase 116 and the creation process is explained in later part of the specification. The one or more pathogenic microbes are identified by mapping pathogenic microbial taxa obtained from the microbial taxa (identified at step 206 of the method 200) to the pathogenic microbial taxa present in the disease microbe map (DM map) of the epitope knowledgebase 116.

Further, at step 210 of the method 200, one or more microbial peptides are identified from the biological sample collected at step 202 of the method 200. The peptide identification module 112 includes one or more microbial peptides identification techniques available in the art for identifying the one or more microbial peptides and microbes present in the biological sample. The microbial proteins/ peptides are also identified in the biological sample by various protein analysis and detection techniques. Phenotypic or molecular techniques are used to quantify the microbes or the microbial peptides from the biological sample. In one embodiment the combination of phenotypic techniques includes microscopic microbial identification, Macroscopic Morphology detection assay, and Phage Typing. Further, the automated D/AST Instrument can be used which largely reduces the time for culture and biochemical testing.

In an embodiment, the identification of multiple microbial components such as cell wall mycotic acid, nucleotides, and peptides in the biological sample, are done through one or more of Serological Tests, Immunoaffinity Chromatography, enzyme-linked immunosorbent assay (ELISA), matrix assisted laser desorption ionization-time of flight mass spectrometry (MALDI-TOF MS), high-performance liquid chromatography (HPLC) methods in a combination thereof. In another embodiment, the culture independent methods of microbial identification techniques include one or more of next generation sequencing procedures such as 16S rRNA sequencing, Whole-Genome Sequencing, mate-pair library, or a paired-end library-based sequences, and Real-Time PCR. The 16srna based sequencing techniques utilizes the identification of marker gene in the sample to detect the taxonomy of the sequence read, while Whole-genome sequencing provides a comprehensive understanding of the entire genome by obtaining the sequencing reads for entire genome. Taxonomic binning, annotation and identification techniques can also be used to identify microbes in the sample. Various techniques are used to segregate sequence reads to taxonomic groups such as supervised and unsupervised classifiers for taxonomic binning of metagenomics and other sequence alignment and similarity tools. The whole proteome or gene identification in sample is done using various protein sequencing techniques and RNA sequencing methods which provide abundant and comprehensive genetic data which can be processed to detect the microbes and microbe specific molecules in the sample.

Further, at step 212 of the method 200, one or more microbial epitopes are identified from the one or more microbial peptides (identified at step 210 of the method 200) present in the biological sample of the subject. The epitope identification module 114 is configured to identify the one or more microbial epitopes from the one or more microbial peptides using the epitope knowledgebase 116.

Further at step 214 of the method 200, the one or more autoimmune disorders such as PBC, AD, and so on, are assessed based on at least one of (i) the one or more pathogenic microbes identified at step 208 of the method 200 and (ii) the one or more microbial epitopes identified at step 212 of the method 200. The disease assessment module 118 is configured to assess the one or more autoimmune disorders of the subject, based on at least one of (i) the one or more pathogenic microbes and (ii) the one or more microbial epitopes, using the epitope knowledgebase 116. The epitope knowledgebase 116 is used to identify the one or more autoimmune disorders by assessing the one or more pathogenic microbes and the one or more microbial epitopes at this step.

As mentioned earlier, the epitope knowledgebase 116 includes the DM_map, the X_RMME_map and the X_RMME_detail_map. The DM_map comprises of the mapping of autoimmune disease to the microbe set. The DM_map provides information about microbes associated with the autoimmune disease 'X' where 'X' is any autoimmune disorder under the scope of the present disclosure. The X_RMME_map comprises mapping of the refined molecular mimic epitopes to a disease 'X'. The epitopes can be identified based on desired autoimmune disorder from the mapping and then identified epitopes can be utilized for the diagnostic and treatment of disease 'X'. The X_RMME_detail_map contains detailed information about the refined epitopes presents in X_RMME_map. The X_RMME_map consists of mapping of human and microbial protein having the mimic epitopes along with the information on the location of the epitope in the protein sequence, microbes which express these epitopes, the protein annotation information, match percentage and if the epitope is MHC binding or antibody binding.

Table 1 shows an exemplary X_RMME_detail_map showing one instance for the autoimmune disorder type: primary biliary cholangitis (PBC):

**Table 1**

| **s n o** | **Ide ntif ier** | **epitope (human )** | **S t a rt** | **e n d** | **human protein info** | **epit ope (mic robe )** | **S t a rt** | **E n d** | **microbe protein info** | **M at ch** | **T y p e** |
|---|---|---|---|---|---|---|---|---|---|---|---|
| 2 | D2 | MTAL | 4 | 4 | sp\|O95342\| | VGP | 3 | 3 | tr\|A0A1A3KPY | 10 | A |
| | | VGPSG | 5 | 6 | ABCBB_H | SGA | 1 | 2 | 1\|A0A1A3KPY | 0 | B |
| | | AGKS | 0 | 2 | UMAN | GK | 7 | 4 | 1_MYCGO | | E |
| | | | | | Bile salt export pump | | | | Cytochrome bd biosynthesis ABC transporter | | |
| | | | | | OS=Homo sapiens | | | | ATP-binding protein | | |
| | | | | | OX=9606 | | | | OS=Mycobacter ium gordonae | | |
| | | | | | GN=ABC | | | | | | |
| | | | | | B11 PE=1 | | | | OX=1778 | | |
| | | | | | SV=2 | | | | GN=A9W97_1 9050 PE=4 | | |
| | | | | | | | | | SV=1 | | |

As shown in Table 1, a disease identifier for the PBC is mentioned as D2. The mentioned instance contains information regarding the mimicry epitope VGPSGAGK identified from cytochrome bd biosynthesis ABC transporter ATP-binding protein with a sequence identifier (ID) A0A1A3KPY1 which is present in *Mycobacterium gordonae.* The epitope VGPSGAGK shows significant sequence similarity to the epitope identified in bile salt export pump protein in human as shown in column 7. The match is 100% and the epitope identified is an antibody binding epitope (ABE) eliciting B cell mediated response. Like the mentioned instance, the X_RMME_detail_map is created for every autoimmune disorder in a similar format for further mapping and information retrieval.

The epitope knowledgebase 116 consists of hash map containing disease identifier as key and microbial mimicry epitopes, microbe from which the mimic epitope is identified, microbial protein in which mimic epitope is present, human epitope, human protein in which epitope is present as subsequent values. The mimicry epitope is a microbial epitope showing significant sequence and structural similarity with human peptide and has the capability to bind to MHC II molecule and represented to the T-cells or can bind to the autoantibodies generated in a particular autoimmune disorder.

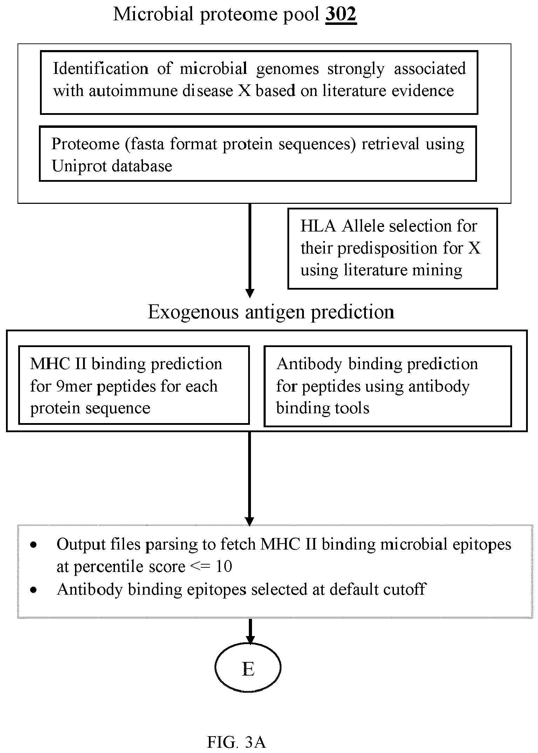

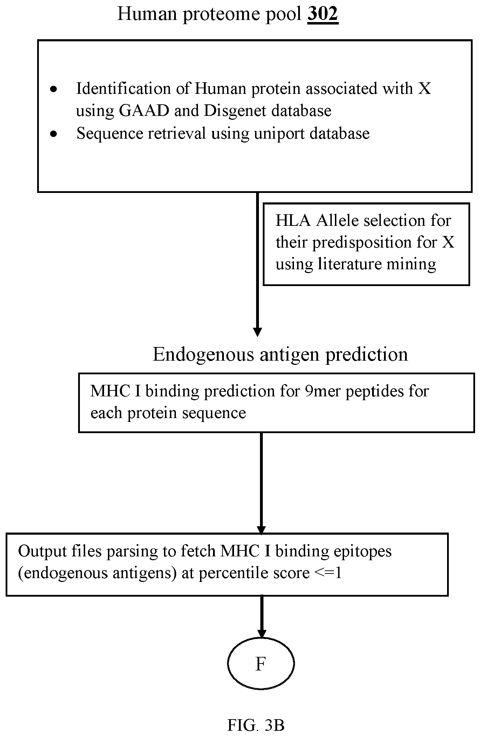

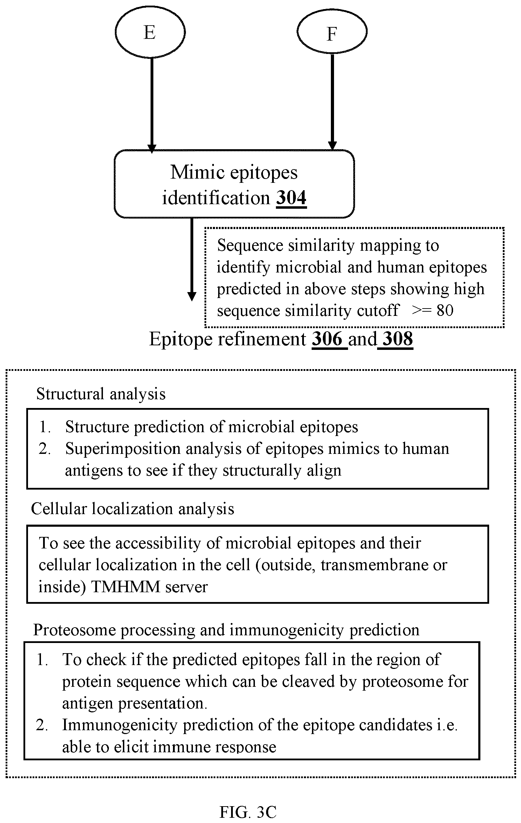

The epitope knowledgebase 116 is created using the knowledge of the different autoimmune disorders and the epitopes present in the art. FIG. 3A through 3C are flowcharts illustrating a process for identifying mimic epitopes that are used for creating the epitope knowledgebase 116, according to some embodiments of the present disclosure. The detailed process for identifying mimic epitopes that are used for creating the epitope knowledgebase 116 is explained in detail through steps 302 to 308 in line with FIG. 3A through 3C.

At step 302, a plurality of disease specific proteomes from both human (human proteome pool) and microbes (microbial proteome pool), are identified that are pertaining to different autoimmune disorders. The different autoimmune disorders are not limited to PBC, atopic dermatitis (AD), psoriasis, other inflammatory disorder or autoimmune condition under the scope of the present disclosure. The plurality of disease specific proteomes is present in the form of matrix called as a microbe disease map and denoted as MD_map. The microbe disease map comprising of a mapping of the autoimmune disorder to the microbes associated with the disorder. In the microbe disease map, each autoimmune disorder is denoted with a unique identifier, for example, D1 and D2 are identifiers for the diseases AD and PBC respectively. The microbes may be directly or indirectly associated with the progression of the autoimmune disease.

In an embodiment, extensive literature mining is used to identify such microbes which are reported to be strongly associated with the disorder. An exemplary keyword search used for the literature mining includes:
1. `X' + microbes
2. 'X' + bacteria + association
3. 'X' + microbiome + association

Table 2 shows an exemplary DM_map.

**Table 2**

| S.no | Disorder | Microbe set |
|---|---|---|
| D1 | Atopic dermatitis | *Staphylococcus aureus MW2, Staphylococcus aureus MRSA252, Staphylococcus haemolyticus (strain JCSC1435, Malassezia restricta CBS 7877, Staphylococcus aureus NCTC 8325* |
| D2 | PBC | *Acinetobacter baumannii ATCC 17978, Chlamydia pneumoniae, Escherichia coli K12, Klebsiella pneumoniae subsp. pneumoniae ATCC 700721, Mycobacterium gordonae, Neisseria meningitidis, Proteus mirabilis HI4320 Staphylococcus aureus NCTC 8325, Novospingobium aromaticovorans* |
| Dm | Disease_x | *Microbe1, mirobe2......* |
| Dn | Disease_y | *Microbe8, microbe9, microbe10,* ........ |
| Do | Disease_z | *Microbe20, microbe30, microbe70......* |
| -- | -- | ------ |

As shown in the Table 2, the proteome for each microbe is mapped to the autoimmune disease type such as Disease_x, Disease_y. and so on. The proteome is the entire protein sequences (fasta format) for a particular microbe derived from the uniprot database. The proteome set is called `MP' which is microbe proteome map comprising of all the protein sequences derived from the microbial genome. The MP for each microbe in 'X' is collectively called 'DMP' which is a disease specific microbial proteome comprising of the collective proteomes of all the microbes in microbe set mapped to disease 'X' in the DM_map.

In one embodiment human proteome is filtered to extract only those protein sequences which are associated with the autoimmune disorder 'X'. This refinement is achieved through one or combination of various techniques such as database search and extensive literature mining. There are gene and autoimmune disorder association databases which identify such mapping through text mining, experimental data, gene expression profiles etc. and provide scoring for such an association. In another embodiment, the protein sequences are extracted in fasta format to form a disease human proteome (DHP) comprising of the human protein sequences specific for a particular autoimmune disorder.

At step 304, the disease specific epitopes in microbes are identified utilizing the proteome set present in the DM_map obtained at step 302. The disease specific epitopes are predicted for each protein sequence in 'DMP' based on the binding capability of these short exogenous peptides to Major histocompatibility complex (MHC) class II molecules and antibodies and `DHP' based on the binding capability of the endogenous peptides from the proteins expressed in humans to MHC class 1 molecules. The Human leukocyte antigen (HLA) allele to which the binding prediction is performed is a disease specific selection based on alleles implicated in different autoimmune disorders. In one embodiment, the selection can be done by automated literature mining to identify such association. The binding of MHC II and the peptide needs to be predicted using techniques known in art. In one embodiment, the peptides of short length can be identified which can act as epitopes capable of eliciting the immune response. In another embodiment, the length of this peptide may be of 9 amino acids. Any other length of peptide predicted through any technique of finding epitopes is in the scope of present disclosure. In another embodiment, the MHC 1 and human peptides binding predictions can be predicted using any technique known in the art to generate shorter peptides which can be a potential autoantigen.

In another embodiment, the antibody binding prediction utilizing any technique known in the art identifies potential microbial and human epitopes in the range from 7mer to 30mer which are capable of binding to antibody and developing B cell immune response. The epitopes are filtered based on scoring cut off to obtain the top predictors. Two epitope pools are obtained from the process. An ExEP is an exogenous epitope pool comprising of the predicted epitopes of microbial origin and the EnEP is an endogenous epitope pool comprising of the epitopes predicted from human proteome pool.

At step 306, the epitopes derived from microbial peptides that show significant sequence similarity with self- peptides and are sufficient to result in the cross-activation of autoreactive T are identified. In an embodiment, the sequence similarity score of greater than 90% is utilized to screen for highly similar mimics. The epitopes mapped at lower sequence similarity cutoff score than 90% and may range from 70% to 90 % which may show similar structural and functional properties in eliciting invariable immunogenicity are also in the scope of present disclosure. The epitopes from ExEP and EnEP are compared for significant sequence similarity to segregate the peptides present in both pools. These epitopes are termed molecular mimic epitopes (MME) which can be potential targets for diagnosis and therapeutics in autoimmune disorder.

At step 308, the potential molecular mimic epitopes obtained at step 306 are further refined based on structure superimpositions to human peptides, immunogenicity of these epitopes and proteasomal cleavage scores in order to be presented to immune cells. The process includes structural superimposition and analysis, cellular localization prediction, proteosome processing and immunogenicity prediction.

### Structural analysis:

In one embodiment, the molecular mimic epitopes MME identified through sequence similarity mapping are screed further for their structural similarity to the corresponding epitopes from human proteins. The protein 3-dimensional (3-D) structure models are created for microbial proteins using structure prediction algorithms and ab initio model creation tools. The epitope regions from microbes and the human proteome are superimposed by considering the flanking regions as well to see if they show structural similarly which increases the confidence for the prediction of the mimic epitopes to generate similar immunogenic response in the body.

### Cellular localization of epitopes:

The cellular localization of epitopes is one of the crucial steps to see if the epitopes predicted in microbes are accessible to the immune cells in the host. In most cases if the epitope lies in the outside region in the cell, it has a high chance of eliciting immunogenic response. So, such epitope candidates are potential mimic candidates which can aggravate the immunogenic response in disease state. In another embodiment, the accessibility of these epitopes and their cellular localization in the cell (outside, transmembrane, or inside) are analyzed to add confidence to the results.

### Proteosome processing and immunogenicity prediction:

In one embodiment, the 'MME' are further checked for their proteasomal cleavage capability because the antigen processing into small peptides or antigens is important for these peptides to be presented to T cell or B cells by antigen presenting cells via their binding to MHC II complex. antigen processing tools predict the proteolytic cleavage residues and if they fall in the epitope regions or not. The scores are used to filter the potential epitopes which show high chances of proteolytic cleavage and presentation to the immune cells.

The Class 1 immunogenicity prediction analysis predicts if the epitope presented on HLA class I molecules is able to elicit T cell immunogenic response. This analysis is important to check if the endogenous epitopes predicted by MHCI binding prediction is able to elicit immunogenic response if presented via APCs. In one embodiment, the endogenous epitopes predicted by MHC II binding prediction methods are accessed for predicting their immunogenicity based on immunogenicity score.

In an embodiment the 'MME' are scanned through Immunogenicity prediction tool which predicts the probability of the exogenous antigens / microbial epitopes to elicit immunogenic response if presented by APCs via MHCII binding mechanism.

Thus, the MMEs are screened and filtered to obtain refined molecular mimicry epitope (RMME) candidates having capability of generating high immune response. The RMME candidates can be used as therapeutic and diagnostic candidates in autoimmune disorders.

The microbial agents extracted in the DNA/RNA extraction at step 204, can be the DNA or mRNA reads specific to the microorganism with the help of which the abundance of any specific microbes can be estimated. In one embodiment, the taxa abundant data obtained through the DNA extraction and sequencing module 110 and such the taxa abundant data is used as input to the epitope knowledgebase 116 where the disease specific taxa mapping is helpful in identifying the autoimmune condition based on the sample.

In another embodiment, the epitope knowledgebase 116 also contains information about the antigens or peptides specifically involved in a respective disease. The peptides or antigen / autoantibodies extracted from the sera sample or infected site can be mapped to the knowledgebase in order to identify the microbial agents involved in the disease pathogenesis. The microbial agents which can be microbes, microbial epitopes or peptides can be extracted from the database pertaining to a particular autoimmune disorder or inflammation and can be utilized in as immunomodulatory composition in order to be administered to subject in need so as to ameliorate disease symptoms.

For example, Table 3 is an exemplary DM_map for PBC where the DM_map is present in the epitope knowledgebase 116:

**Table 3**

| S.no | Disorder | Microbe set |
|---|---|---|
| D2 | PBC | *Acinetobacter baumannii ATCC 17978, Chlamydia pneumoniae, Escherichia coli K12, Klebsiella pneumoniae subsp. pneumoniae ATCC 700721, Mycobacterium gordonae, Neisseria meningitidis, Proteus mirabilis HI4320, Staphylococcus aureus NCTC 8325, Novospingobium aromaticovorans* |

If the infected sample contains an abundance of any one or more combination of microbes listed in the Table 3 under column Microbe set in the row D2 which represent PBC then it is likely that these microbes are involved in the exacerbation of the biliary infection and inflammation.

Table 4 shows the exemplary RMME comprising of microbial epitopes identified for PBC:

**Table 4**

| Sequence ID | Epitope sequence | Disease | MHC II / antibody binding |
|---|---|---|---|
| D2_1 | LVGPSGAGKS | PBC | ABE |
| D2_2 | VGPSGAGK | PBC | ABE |
| D2_3 | PLTGASMNPARSFG | PBC | ABE |
| D2_4 | SIPENEPGS SIMPGKVNPTQC | PBC | ABE |
| D2_5 | AIPENEPGS SIMPGKVNPTQC | PBC | ABE |
| D2_6 | KIPENEPGS SIMPGKVNPTQC | PBC | ABE |
| D2_7 | FPENEPGS SIMPGKVNPTQC | PBC | ABE |
| D2_8 | TSALDTESERA | PBC | ABE |
| D2_9 | IVIAHRLST | PBC | MHCII |
| D2_10 | VIAHRLSTI | PBC | MHCII |
| D2_11 | LMLVTALNP | PBC | MHCII |
| D2_12 | IVIAHRLST | PBC | MHC II |
| D2_13 | VIAHRLSTI | PBC | MHC II |
| D2_14 | LMLVTALNP | PBC | MHC II |
| D2_15 | TIVIAHRLS | PBC | MHC II |
| D2_16 | RIAIARALV | PBC | MHC II |

In one embodiment, the disease once identified in the DM_map is mapped in the 'X_RMME' which comprises disease specific RMMEs (as shown in Table 4). The mapping leads to extraction of PBC specific microbial epitopes which may be implicated in the disease. The sequence identifiers (IDs) D2_1 to D2_16 in Table 4 represents the 'D2_RMME' that are identified as mimicry peptides/epitopes implicated in PBC. In another embodiment, these microbial agents can be utilized in combinations and in laboratory acceptable procedures to be administered to the individual.

Table 5 shows the exemplary RMME comprising of microbial epitopes identified for AD. As shown in the Table 5, a list of mimic epitopes from sequence ID D1_1 to D1_35 for the treatment of AD is specified and the list is called 'D1_RMME'.

**Table 5**

| Sequence ID | Epitope Sequence | Disease | MHC II / antibody |
|---|---|---|---|
| | | | binding epitopes |
| D1_1 | VQKTPQGRSFVRPSGTED | Atopic Dermatitis | ABE |
| D1_2 | DWNPQNDLQ | Atopic Dermatitis | ABE |
| D1_3 | PGHVDFSSEVS | Atopic Dermatitis | ABE |
| D1_4 | YPQYSCSTTGSS | Atopic Dermatitis | ABE |
| D1_5 | ITPVPGGVGP | Atopic Dermatitis | ABE |
| D1_6 | ITPTPLGEGKSTTT | Atopic Dermatitis | ABE |
| D1_7 | RQPSQGPTFGIKGGAAGGGYS QV | Atopic Dermatitis | ABE |
| D1_8 | PDICKDYKET | Atopic Dermatitis | ABE |
| D1_9 | DWNPHQDLQAQDRAHR | Atopic Dermatitis | ABE |
| D1_10 | GSRRQEAQ | Atopic Dermatitis | ABE |
| D1_11 | TAGQEDYDRL | Atopic Dermatitis | ABE |
| D1_12 | EMPSGTGK | Atopic Dermatitis | ABE |
| D1_13 | QWGDEGKGK | Atopic Dermatitis | ABE |
| D1_14 | DEPTTNLDRE | Atopic Dermatitis | ABE |
| D1_15 | TLSEESYK | Atopic Dermatitis | ABE |
| D1_16 | SGAGNNWAKGHYTEGAE | Atopic Dermatitis | ABE |
| D1_17 | GEGMDEMEFTEAES | Atopic Dermatitis | ABE |
| D1_18 | ANEAKGTKVV | Atopic Dermatitis | ABE |
| D1_19 | DSAGQKGTGKWT | Atopic Dermatitis | ABE |
| D1_20 | ITELPPTHP | Atopic Dermatitis | ABE |
| D1_21 | VPSGASTGVH | Atopic Dermatitis | ABE |
| D1_22 | RSGETEDTTI | Atopic Dermatitis | ABE |
| D1_23 | GAITPVPGGVGP | Atopic Dermatitis | ABE |
| D1_24 | LDSRGNPTV | Atopic Dermatitis | ABE |
| D1_25 | VPSGASTGEHEAVELRDGDKS RY | Atopic Dermatitis | ABE |
| D1_26 | HRSGETEDTTI | Atopic Dermatitis | ABE |
| D1_27 | LVGPSGAGKS | Atopic Dermatitis | ABE |
| D1_28 | VPSGASTGEHEAVELRDGDKS RYSGKGVTKAVE | Atopic Dermatitis | ABE |
| D1_29 | NVAIVGPSGAGKT | Atopic Dermatitis | ABE |
| D1_30 | KLEKERQE | Atopic Dermatitis | ABE |
| D1_31 | ATLSVHQLV | Atopic Dermatitis | MHC II |
| D1_32 | LRFLRYGSL | Atopic Dermatitis | MHC II |
| D1_33 | QRVAIARTI | Atopic Dermatitis | MHC II |
| D1_34 | RITLTSRNV | Atopic Dermatitis | MHC II |
| D1_35 | SRGNPTVEV | Atopic Dermatitis | MHC II |

Disease assessment and diagnostic techniques: Various techniques are utilized to assess the individual for a particular autoimmune disorder, in the context of the present disclosure.

The methods and systems of the present disclosure identify disease specific epitopes which can be detected in the collected biological sample using the epitope knowledgebase 116. In one embodiment, the set of disease specific mimicry epitopes set which is 'RMME' termed as refined molecular mimicry epitopes set extracted from the epitope knowledgebase 116 can be utilized in one or more combinations to diagnose autoimmune disorder or similar inflammatory conditions in patients. Post sample collection, the molecular agents are extracted from the collected sample which can be blood sample, sera sample or any other body fluid extracted from the site of infection.in another embodiment the molecular agents can be DNA or RNA fragments, short peptides, proteins, antigen -antibody titers extracted from the sample and the microbial agents can belong to both human and microbes.

The extracted microbial epitopes which are short antigenic peptides mentioned as one of the molecular agents can be mapped to the epitope knowledgebase 116. The epitope knowledgebase 116 contains information about the antigens or peptides specifically involved in a respective disease. The peptides or antigen / autoantibodies extracted from the sera sample or infected site pertaining to human or microbial origin can be mapped to the knowledgebase in order to identify the autoimmune disorder or any inflammatory condition leading to the diagnosis of the disease.

In one embodiment, the epitopes or small peptides from the infected sample can be identified and sequenced using various methods known to one skilled in the art. One or more of the combinations of various methods for the extraction, identification, and screening of such small peptides and epitopes robustly is crucial for the accurate diagnosis of the disorder. The combination techniques such as Mass spectrometry with surface plasmon resonance (SPR) biosensor can be used for accurate identification and affinity determination of peptide antibody interaction while eluted epitopes in SPR chip can be later identified using MALDI-MS. Other methods such as peptide chip analysis in combination with mass spectrometry, peptide microarray screening, Tricine-SDS-PAGE using electrophoretic system, whole peptide mapping can efficiently and in cost effective way detect the microbial and human based small peptide/epitope fragments in the sample.

The shorter peptide or epitope sequences obtained from the above methods are then mapped to the disease specific epitopes or peptides captured in the knowledgebase. one or more combination of ways can be utilized to map and identify if the sample containing the epitopes are present in the database or not such as sequence mapping to obtain similarity score for the epitopes detected, if the similarity score is significant then the disorder is present. One or more combinations of epitopes can be utilized for the diagnostics.

The sera from the infected area can be made to react with the disease specific RMME set. The epitopes from each RMME set can be synthesis using peptide synthesis methods for peptide synthesis and these short peptides can be placed in microarray chip and made to interact with the sample sera containing antibodies against the antigens in the disease state. The detection can be done using any enzyme assay-based detection which includes the emission of signals when the antibodies in the sera react to the epitopes present in the microarray chip post antigen-antibody interaction. If the detection signals are recorded, and then the disorder can be diagnosed accurately.

The methods and systems of the present disclosure identify microbial epitopes or peptides which show significant molecular mimicry to human proteins. The set of such mimicry epitopes called `RMME' refined molecular mimicry epitopes listed here can be utilized for early diagnosis to combat autoimmune disorders or any inflammatory conditions under the scope of present disclosure. In one embodiment, the RMME set identified for PBC can be detected in the collected samples from blood, sera or any other infected fluids taken from the subject.

The sequences from sequence ID D2_1 to D2_16 from Table 4, can be utilized in one or more than one combination to diagnose the PBC in infected patents by detecting binding of antibodies present in the sera to the epitope sequences in the epitope knowledgebase 116. In yet another embodiment, the sequences from sequence ID D2_1 to D2_16 can be enzyme labelled and can be fixed to a microarray chip and is washed by the sera antibodies and the hybridization signals can be captured and diagnosis can be done. While in other scenarios the antibodies extracted from the sera sample can be enzyme labelled for signal detection and fixed in the array chip and is washed by synthetically produced sequences from sequence ID D2_1 to D2_16 to check for hybridization signal.

In one embodiment the RMME set identified for PBC can be detected in the collected samples from blood, sera or any other infected fluids taken from the subject. In an embodiment, the sequence IDs D1_1 to D1_35 in Table 5 is utilized for the treatment of AD. In another embodiment, the sequences from sequence ID D1_1 to D1_35 can be utilized in one or more than one combination to diagnose the AD in infected patents by detecting binding of antibodies present in the sera to the epitope sequences in the knowledgebase. In yet another embodiment, the sequences from sequence ID D1_1 to D1_35 can enzyme labelled and can be fixed to a microarray chip and is washed by the sera antibodies and the hybridization signals can be captured and diagnosis can be done. While in other scenario the antibodies extracted from the sera sample can be enzyme labelled for signal detection and fixed in the array chip and is washed by synthetically produced sequences from sequence ID D1_1 to D1_35 to check for hybridization signal.

The methodologies described are not limited to PBC and AD but to any other autoimmune disorders and inflammatory conditions are under the scope of present disclosure.

Further at step 216 of the method 200, an artificial sequence construct is generated using one or more mimic epitopes identified from the epitope knowledgebase 116 through the sequence construct generation module 120. The artificial sequence construct comprises of one or more refined molecular mimic epitopes associated with the one or more autoimmune disorders present in the epitope knowledgebase 116, linked by (i) one or more peptide linkers, (ii) one or more adjuvants, and (iii) one or more toll-like receptor (TLR) ligands, to enhance an immunogenicity. The artificial sequence construct is generated to administer the subject for the one or more autoimmune diseases assessed at step 214 of the method 200.

Various techniques are employed to administer microbial agents to the subject or at the infected area. The technique of administration depends on the disease condition, site of infection. In an embodiment, the administration techniques can be utilized to introduce microbial agents to the subject, including but are not limited to intradermal, intramuscular, intraperitoneal, intravenous, subcutaneous, oral, ophthalmic, rectal, topical, or pulmonary, and intranasal routes.

In one embodiment, the microbial modulation in the form of microbial cocktails, mimic epitopes, microbial peptides can be utilized in number of ways as a potential therapy to treat autoimmune conditions. The microbe-based therapies overcome the limitations of existing treatments as they can be designed as personalized therapies which improve the efficacy of the treatment. The prebiotic and probiotic treatments are disease specific so pose less side effects and are efficacious. Diet-based therapies can be safer and effective treatment strategies which can have a stronger impact on gut microbiome in context of microbial association with autoimmune disease.

The mimic epitopes identified for specific disease can be a potential vaccine candidate or antigenic peptides which can be administered orally or topically (in case of skin related autoimmune disorders). Skin patch to administer vaccines are needle-free and non-invasive which may reduce medical costs by allowing personnel with a lower level of medical training to administer them. Transcutaneous immunization is a safe and effective strategy for inducing strong mucosal antibody and CTL responses offering strong tolerization because antigens expressed in the epidermis are more immunogenic due to a sensitive immune surveillance mechanism in the skin. In yet another embodiment, the mimic epitopes can be utilized to generate monoclonal antibodies which can be helpful in neutralizing autoantigens as well as mimic epitopes exacerbating immune response.

The methods and systems of the present disclosure identify microbial epitopes/peptides which show significant molecular mimicry to human proteins for the treatment of autoimmune disorders or any other inflammatory conditions. In one embodiment, the set of such mimicry epitopes called 'RMME' the refined molecular mimicry epitopes listed here can be utilized as an alternative treatment to combat PBC. The methodology is not limited to PBC and AD but to any other autoimmune disorders and inflammatory conditions under the scope of present disclosure.

In another embodiment, the sequences from sequence ID D2_1 to D2_16 from Table 4 can be utilized in one or more than one combination to treat PBC. In yet another embodiment, the sequence IDs D2_1 to D2_16 are short peptides ranging from 7 to 30 amino acid residues which can be delivered through various administration routes as potential vaccine candidates also called tolerizers autoantigens or bystander antigens for inducing tolerization of immune system. In another embodiment, tolerization is not limited to oral, nasal, or transcutaneous immunization route but any other delivery method known to those skilled in the art.

In one embodiment, the construct comprises of the combination of the epitope sequences from sequence ID D2_1 to D2_16 which are specific for the PBC which are linked by linker peptides and the adjuvant to make a synthetic construct. In another embodiment, the construct comprises of the combination of the epitope sequences from sequence ID D2_1 to D2_16 which are specific for the PBC which are linked by linker peptides to form immunogen fused along with the TLR ligand to form fusion construct. In another embodiment, the B-cell, CTL, and HTL epitopes are selected from the list of epitope sequences from sequence ID D2_1 to D2_16 for PBC to create a multivalent immunogen capable of imparting tolerization in the autoimmune conditions. The epitopes in the above embodiment are linked by a suitable linker peptide to create a fusion peptide construct. The peptide linker can be GGGS, AAY, KK, GPGPG, HEYGAEALERAG where the B-cell epitopes and HTL epitopes (MHC II epitopes) can be linked by KK and GPGPG linker. The adjuvant can be fused to the construct with the help of EAAAK linker and to enhance the stability and the efficacy of the construct. The adjuvants can be used for two broad purposes based on mechanism of their action mainly vaccine delivery and immunostimulatory adjuvants. The combination of the adjuvants can be used for efficient delivery of the vaccine construct to the site of action and enhancing the immunogenicity of the construct as well. The adjuvant used as vaccine delivery can be any particulate emulsions, microparticles, iscoms cochleates and liposomes which can target the attached epitopes into antigen presenting cells (APC), including macrophages and dendritic cells. Also, various plant-based proteins such as lectins and bacterial proteins such as CTB or LTB which have high binding affinity for epithelial surfaces can be used for the delivery of the antigen. In another embodiment, the natural adjuvants can be used such as IL-1 Mast cell activators, Vitamins: vitamin A (retinoic acid), vitamin D₃, and vitamin E which are safer to use The adjuvant also comprise of the immunostimulatory molecules which can be one of the immunogenic peptides derived from the pathogens but is nontoxic to the subject and often represent pathogen associated molecular patterns (PAMP) e.g., LPS, MPL, CpG DNA, which can activate cells of the innate immune system. The synthetic vaccine construct can be delivered through sesicular carrier systems, like liposomes, vesosomes, niosomes and transferosomes, which is the most favorable carrier system for topical delivery of vaccines. In the case of synthetic vaccine construct comprising of TLR ligand which can activate APCs and dendritic cells where the TLR ligand is conjugated with the fusion peptide construct comprising of the epitopes linked by the linker peptides. Any one of the TLR ligand as mucosal adjuvant can be utilized for enhanced immunogenic response such as MPL (TLR2 and TLR4 ligands) CpG ODN (TLR9 ligand), CTB-CpG, Flagellin (TLR5 ligand).

In one embodiment, the sequences D2_1 to D2_16 can be administered as immunomodulating composition in any combination of peptide sequence listed in Table 4 based on how efficacious the sequences are for the treatment. In another embodiment, these microbial agents can be conjugated with immunogenic carrier proteins in order to enhance their immunogenicity and efficacy. Most common immunogenic carriers include but are not limited to Limulus polyphemus hemocyanin (LPH), Tachypleus tridentatus hemocyanin (TTH), and bovine serum albumin (BSA), and nanoparticle formulations etc.

In another embodiment, the sequences IDs D2_1 to D2_16 can be utilized to identify other microbial candidates which may be involved in disease exacerbation. The proteome of other microbial candidates can be scanned for the presence of the sequence IDs D2_1 to D2_16. In cases where a particular microbe contains a protein with any of the above listed sequence IDs then that microbe might be inducer of molecular mimicry in PBC condition. In another embodiment various methods such as antimicrobials and dietary shift treatments can be utilized to reduce the abundance of such microbes in the microbiome in order to combat disease.

In one embodiment the sequences IDs D2_1 to D2_16 can be used in combination to generate monoclonal antibodies peptide-specific MHC-restricted monoclonal antibody which can be administered to the individual. These monoclonal antibodies can be utilized to neutralize the immune response in the PBC. The sequence IDs D2_1 to D2_16 can be exploited to generate monoclonal antibodies under standard laboratory procedures and can be delivered in a way which is medically acceptable. In yet another embodiment, these monoclonal antibodies can be designed in a way specific to only microbial epitopes but not showing cross reactivity to self-antigens. There may be various processes to generate such microbial epitope specific monoclonal antibodies such as immunization of transgenic mice with soluble peptide/MHC complexes.

The monoclonal antibodies can be generated by any one of the techniques such as hybridoma technology using any one of the cell lines Chinese hamster ovary (CHO) cells, Human embryonic kidney (HEK293) cells, Baby hamster kidney (BHK-21) cells, Mouse myeloma (NS0) cells, phage display technology, single B cell culture, single cell amplification from various B-cell populations, single plasma cell interrogation technologies, recombinant monoclonal antibodies production involving repertoire cloning, CRISPR/Cas9, or phage display/yeast display technologies which one skilled in the art can use for the production of these antibodies. The monoclonal antibodies produced against the epitopes mentioned in above embodiments can be mass produced through one of the conventional technologies such as clonal expansion using selective media, bioreactors, or large flasks. The monoclonal antibodies can be purified by any one of the various purification techniques such as chromatography techniques, protein A/G affinity chromatography, affinity purification systems, high-throughput metal-chelate methacrylate monolithic system. The purified monoclonal antibody specific for the mimic epitopes can be administered to the subject in a pharmaceutical composition comprising of the monoclonal antibody and a pharmaceutical carrier.

In yet another embodiment, the microbial agents such as sequence ID D2_1 to D2_16 can also be administered to combat PBC, by incubating them in dendritic cells or any other antigen presenting cells under standard laboratory procedures by those skilled in the art. These antigen presenting cells can take up the microbial peptide and can facilitate presentation of MHC epitope(s) present in those peptides. The dendritic or other cells employed for this process can be isolated from the individual to whom they are to be delivered after incubation with the microbial agent.

In one embodiment the set of such mimicry epitopes called `RMME' refined molecular mimicry epitopes listed here can be utilized as an alternative treatment to combat Atopic dermatitis. In another embodiment, immunomodulating composition comprising of sequence IDs D1_1 to D1_35 from Table 5 in one or more than one combination can be administered to the subject in need of the treatment of Atopic dermatitis condition. In yet another embodiment the combinations of peptide sequences with sequence IDs D1_1 to D1_35 can be administered by various ways through oral, mucosal, topical routes. In case of skin related disorders such as Atopic dermatitis oral and topic application of microbial agents can be an effective and efficacious way to subside the inflammation. In another embodiment skin patch can be used to deliver vaccine topically carrying any combination of sequences listed in Table 5 along with conjugate. In yet another embodiment the conjugate can be any immunogenic carrier protein including but is not limited to Limulus polyphemus hemocyanin (LPH), Tachypleus tridentatus hemocyanin (TTH), and bovine serum albumin (BSA), and nanoparticle formulations etc.

In one embodiment the construct comprises of the combination of the epitope sequences from sequence ID D1_1 to D1_35 which are specific for the AD which are linked by linker peptides and the adjuvant to make a synthetic construct. In another embodiment, the construct will comprise of the combination of the epitope sequences from sequence ID D1_1 to D1_35 which are specific for the AD which are linked by linker peptides to form immunogen fused along with the TLR ligand to form fusion construct.

The B-Cell, CTL, and HTL epitopes are selected from the list of epitope sequences from sequence ID D1_1 to D1_35 for AD to create a multivalent immunogen capable of imparting tolerization in the said autoimmune conditions. The epitopes in the above embodiment are linked by a suitable linker peptide to create a fusion peptide construct. The peptide linker can be GGGS, AAY, KK, GPGPG, HEYGAEALERAG where the B-cell epitopes and HTL epitopes (MHC II epitopes) can be linked by KK and GPGPG linker. The adjuvant can be fused to the construct with the help of EAAAK linker and to enhance the stability of the construct. The adjuvants can be used for two broad purposes based on mechanism of their action mainly vaccine delivery systems and immunostimulatory adjuvants. The combination of the adjuvants can be used for efficient delivery of the vaccine construct to the site of action and enhancing the Immunogenicity of the construct as well. The adjuvant used as vaccine delivery system can be any particulate emulsions, microparticles, iscoms cochleates and liposomes which can target the attached epitopes into antigen presenting cells (APC), including macrophages and dendritic cells. Also, various plant-based proteins such as lectins and bacterial proteins such as CTB or LTB which have high binding affinity for epithelial surfaces can be used for the delivery of the antigen. In another embodiment, the natural adjuvants can be used such as IL-1 Mast cell activators, Vitamins: Vitamin A (retinoic acid), Vitamin D₃, and Vitamin E which are safer to use. The adjuvant also comprise of the immunostimulatory molecules which can be one of the immunogenic peptides derived from the pathogens but is nontoxic to the subject and often represent pathogen associated molecular patterns (PAMP) e.g., LPS, MPL, CpG DNA, which can activate cells of the innate immune system. The synthetic vaccine construct can be delivered through Vesicular carrier systems, like liposomes, vesosomes, niosomes and transferosomes, which is the most favorable carrier system for topical delivery of vaccines. In the case of synthetic vaccine construct comprising of TLR ligand which can activate APCs and dendritic cells where the TLR ligand is conjugated with the fusion peptide construct comprising of the epitopes linked by the linker peptides. Any one of the TLR ligand as mucosal adjuvant can be utilized for enhanced immunogenic response such as MPL (TLR2 and TLR4 ligands) CpG ODN (TLR9 ligand), CTB-CpG, Flagellin (TLR5 ligand).

In one embodiment the sequence IDs D1_1 to D1_35 present in Table 5 can be used in one or more combinations to generate monoclonal antibodies which bind to MHC-microbial peptide complex and neutralize the immune response in Atopic dermatitis patients. These monoclonal antibodies are designed in a way specific to only microbial epitopes but not showing cross reactivity to self-antigens. In another embodiment the microbial epitope specific monoclonal antibodies can be generated by various methods which are known to the person skilled in the art such as immunization of transgenic mice with soluble peptide/MHC complexes. In yet another embodiment the monoclonal antibodies specific to the microbial peptides containing the epitopes form sequence IDs D1_1 to D1_35 in treating atopic dermatitis can be administered by methods including but not limited to intracranial, intrathecal, intradermal, intramuscular, intraperitoneal, intravenous, subcutaneous, oral, and intranasal routes based on the site of infection and disease condition.

The monoclonal antibodies can be generated by any one of the mention methods such as hybridoma technology using any one of the cell lines Chinese hamster ovary (CHO) cells, Human embryonic kidney (HEK293) cells, Baby hamster kidney (BHK-21) cells, Mouse myeloma (NS0) cells, phage display technology, single B cell culture, single cell amplification from various B cell populations, single plasma cell interrogation technologies, recombinant monoclonal antibodies production involving repertoire cloning, CRISPR/Cas9,or phage display/yeast display technologies which one skilled in the art can use for the production of these antibodies. The monoclonal antibodies produced against the epitopes mentioned in above embodiments can be mass produced via any standard technology one skilled in the art such as clonal expansion using selective media, bioreactors, or large flasks. The monoclonal antibodies can be purified by any one of the various purification techniques such as chromatography techniques, protein A/G affinity chromatography, affinity purification systems, high-throughput metal-chelate methacrylate monolithic system. The purified monoclonal antibody specific for the mimic epitopes can be administered to the subject in a pharmaceutical composition comprising of the monoclonal antibody and a pharmaceutical carrier.

Further at step 218 of the method 200, the efficacy of the artificial sequence construct or the other administering procedures generated to the subject at step 216 of the method 200 are assessed through the efficacy assessment module 122. According to an embodiment of the disclosure, the efficacy assessment module 122 comprises of laboratory acceptable procedures of detecting presence of microbial agents such as antigens, microbes at the infected site in the given autoimmune condition. The procedures in the efficacy assessment module 122 are used to check if the immune response is supressed by the treatment procedure in the infected area.

In one embodiment, the presence of microbe in the infected region can be accessed by techniques such as microscopic imaging of sample, mRNA-based detection of viable microbial cells, any peptide marker specific for the specific microbe can be used to quantify it in the infected site. For example, an antimicrobial peptide (AMP)-based colorimetric bioassay can be used for rapid and sensitive detection of Escherichia coli O157: H7. The antimicrobial peptide-horseradish peroxidase (AMP-HRP) can anchor on the surface of target bacteria rapidly through electrostatic and hydrophobic interactions AMP and horseradish peroxidase (AMP-HRP) conjugate is utilized to develop a signal reporter which can be detected by various detection and signal processing techniques. The mRNA corresponding to expression of these genes can be detected using techniques like but not limited to polymerase chain reaction (RT-PCR) assays or reverse transcriptase strand displacement amplification (RT-SDA) assays.

In another embodiment the 16s RNA amplification based specific techniques for the detection of microbe can be utilized to check if the microbe is still present in the sample. For example, S. aureus can be detected in the sample using in situ hybridization technique with fluorescence-labelled oligonucleotide probes specific for staphylococcal 16S rRNA. In yet another embodiment, specific metabolites or compounds produced by a microbes can also be detected using various laboratory acceptable techniques like Mass spectrometry, HPLC-MS, spectrometry-based methods etc. In case of culturable bacteria, the viability of microbe can even be established using culturing procedures based on selective media followed by techniques to detect specific.

In one embodiment, the efficacy of the treatment can also be examined by observing various phenotypic characteristics such as monitoring the symptoms, detected the antigen antibody titters in the sera. For example, the detection of serum alkaline phosphatase levels in the case of PBC and in case of AD, the skin can be observed for coloration, pigmentations, itchiness, dryness etc. Any other procedure of detecting pathogens is also within scope of this disclosure. The efficacy is accessed and following the administration process offering treatment in combinations until the symptoms are subside and there is no infection detected in the infected area or the sera. Case studies: The following case studies for two diseases the primary biliary cholangitis (PBC) and the atopic dermatitis (AD) are intended to illustrate but not to limit the present disclosure in any manner, shape, or form, either explicitly or implicitly.

### Primary biliary cholangitis (PBC):

PBC is a chronic, autoimmune and cholestatic liver disease characterized by the gradual progressive destruction of intrahepatic biliary ductulus leading to hepatic fibrosis and liver failure. PBC patients constitute 11% of all patients undergoing liver transplantation for cholangitis. PBC is mainly diagnosed in women, with a female: male ratio of about 10:1. The various factors such as genetic risk, dysregulated mucosal immunity, altered biliary epithelial cell function and epigenetic changes lead to the disease pathology and progression. There is a strong association of PBC with other autoimmune disorders such as autoimmune hepatitis (AIH), thyroid dysfunction, sicca symptoms, Raynaud's syndrome, systemic lupus erythematosus (SLE) and rheumatoid arthritis. PBC leads to significant reduction in the quality of life of patients with various symptoms such as pruritus or generalized itching, jaundice, debilitating fatigue which can be observed in almost 80% cases, bone and joint pains, abdominal pains and dry eyes and dry mouth from kerato-conjunctivitis sicca. portal hypertension, esophageal varices, hepatic encephalopathy and osteoma Lacia are some of the complications associated with the progression of primary biliary cholangitis.

Current diagnosis is by routine blood test which detects elevated levels of alkaline phosphatase and blood cholesterol. The serological test targeting anti mitochondrial antibodies is confirmatory in diagnosing the disease. The current treatment used for PBC is with Urso deoxycholic acid (ursodiol), a natural bile acid that is not toxic to the liver, to replace the bile acids and other immune suppressions basically to suppress immune response. Infectious and environmental agents have been proposed as immunological triggers for PBC. Bacterial species such as *Escherichia coli, Novosphingobium aromaticivorans, proteous mirabils,* etc. are associated with the pathology of the disease in numerous studies. Secondary infections caused by these bacteria and molecular mimicry which is similarities between foreign and self-peptides favoring the activation of autoreactive T or B cells by a foreign-derived antigen in a susceptible individual are suggested as important mechanisms to explain microbial implication in PBC autoantibodies are generated against mitochondrial antigen generated from protein PDC (pyruvate dehydrogenase complex) which is involved in the aerobic cellular respiration process and is extremely vital for cell survival.

It is proposed that the primary infection leads to cell apoptosis leading to the release of mitochondrial and other cellular components in the extracellular matrix. These mitochondrial and other self-peptides are exposed to the immune system which attack these self-agents which show considerable molecular mimicry to the microbial peptides against which the antibodies and T cell response is already generated. For example, human PDC-E2 shares a significant homology with *E. coli* PDC-E2 in the region of immunodominant epitope of AMA which leads to breaking of tolerance to mitochondrial autoantigens.

This mechanism is exploited in the present disclosure to identify many such cross-reactive molecular mimics in microbial system which have the ability to generate immune response which can lead to generation of antibodies and MHC binding T Cell response which attack the self-antigens. The identification of many such microbial agents which show molecular mimicry with human peptides is imperative in designing novel and alternate treatment strategies for the disease. Extensive literature mining revealed certain bacteria species in the gut, urinary tract region and duodenum area strongly associated with the PBC progression. The results boiled down to selecting *Acinetobacter baumannii, ATCC 17978, Chlamydia pneumoniae, Escherichia coli K12, Klebsiella pneumoniae subsp. pneumoniae ATCC 700721, Mycobacterium gordonae, Neisseria meningitidis, Proteus mirabilis HI4320, Staphylococcus aureus NCTC 8325, and Novospingobium aromaticovorans* as predominant species for further analysis. The mapping is provided in the DM_map from Table 2.

The proteome of these microbial species is run through epitope identification which generate epitope set termed as ExEP which is exogenous epitope pool comprising of the predicted epitopes of microbial origin as described in Epitope identification. The ExEP was compared to the EnEP comprising the epitopes set predicted for human peptides known to be implicated in PBC. The mapping results were refined to generate `RMME' comprising of disease specific epitope set which show molecular mimicry in our in-silico analysis. The list of potential RMME is provided in the Table 4 and Table 5 for PBC and AD respectively.

In one embodiment the listed sequence IDs D2_1 to D2_16 from Table 4 can be used as immunomodulating composition capable of modulating the immune system in order to subside the symptoms of the disease. Further, D2_RMME_detail_map is created which comprises list of microbial epitopes implicated in PBC and are specified in Table 6:

**Table 6**

| sn o | epitope (huma n) | sta rt | En d | human protein info | epito pe (micr obe) | sta rt | En d | microbe protein info | mat ch | type |
|---|---|---|---|---|---|---|---|---|---|---|
| 1 | MTAL VGPS GAGK S | 45 0 | 46 2 | sp\|O95342\| ABCBB_ HUMAN Bile salt export pump OS=Homo sapiens OX=9606 GN=ABC B11 PE=1 SV=2 | LVG PSG AGK S | 35 5 | 36 4 | tr\|Q2G0B3\|Q2 G0B3_STAA 8 Uncharacteriz ed protein OS=Staphyloc occus aureus (strain NCTC 8325 / PS 47) OX=93061 GN=SAOUH SC_00692 PE=4 SV=1 | 100 | AB E |
| 2 | MTAL VGPS GAGK S | 45 0 | 46 2 | sp\|O95342\| ABCBB_ HUMAN Bile salt export pump OS=Homo sapiens OX=9606 GN=ABC B11 PE=1 SV=2 | VGP SGA GK | 31 7 | 32 4 | tr\|A0A1A3KP Y1\|A0A1A3K PY1_MYCG O Cytochrome bd biosynthesis ABC transporter ATP-binding protein OS=Mycobact erium gordonae OX=1778 GN=A9W97_ 19050 PE=4 SV=1 | 100 | AB E |
| 3 | GASM NPAR SF | 20 9 | 21 8 | sp\|P55087\| AQP4_HU MAN Aquaporin -4 OS=Homo sapiens OX=9606 GN=AQP4 PE=1 SV=2 | PLT GAS MNP ARS FG | 79 | 92 | tr\|A6TI87\|A6 TI87_KLEP7 Uncharacteriz ed protein OS=Klebsiell a pneumoniae subsp. pneumoniae (strain ATCC 700721 / MGH 78578) OX=272620 GN=KPN_pK PN3p05929 PE=3 SV=1 | 100 | AB E |
| 4 | LPEN EPGSS IMPG KVNP TQC | 35 8 | 37 7 | sp\|P07954\| FUMH_H UMAN Fumarate hydratase, mitochond rial OS=Homo sapiens OX=9606 GN=FH PE=1 SV=3 | SIPE NEP GSSI MPG KVN PTQ C | 31 0 | 33 0 | tr\|B4EVY7\|B 4EVY7 PRO MH Fumarate hydratase class II OS=Proteus mirabilis (strain HI4320) OX=529507 GN=fumC PE=3 SV=1 | 95 | AB E |
| 5 | LPEN EPGSS IMPG KVNP TQC | 35 8 | 37 7 | sp\|P07954\| FUMH_H UMAN Fumarate hydratase, mitochond rial OS=Homo sapiens OX=9606 GN=FH PE=1 SV=3 | AIPE NEP GSSI MPG KVN PTQ C | 31 0 | 33 0 | tr\|A6T8M7\|A 6T8M7 KLE P7 Fumarate hydratase class II OS=Klebsiell a pneumoniae subsp. pneumoniae (strain ATCC 700721 / MGH 78578) OX=272620 GN=fumC PE=3 SV=1 | 95 | AB E |
| 6 | LPEN EPGSS IMPG KVNP TQC | 35 8 | 37 7 | sp\|P07954\| FUMH_H UMAN Fumarate hydratase, mitochond rial OS=Homo sapiens OX=9606 GN=FH PE=1 SV=3 | SIPE NEP GSSI MPG KVN PTQ C | 31 0 | 33 0 | sp\|P05042\|FU MC_ECOLI Fumarate hydratase class II OS=Escherich ia coli (strain K12) OX=83333 GN=fumC PE=1 SV=1 | 95 | AB E |
| 7 | LPEN EPGSS IMPG KVNP TQC | 35 8 | 37 7 | sp\|P07954\| FUMH_H UMAN Fumarate hydratase, mitochond rial OS=Homosapiens OX=9606 GN=FH PE=1 SV=3 | KIPE NEP GSSI MPG KVN PTQ C | 30 9 | 32 9 | tr\|Q9JYR9\|Q9 JYR9 NEIM B Fumarate hydratase class II OS=Neisseria meningitidis serogroup B (strain MC58) OX=122586 GN=fumC PE=3 SV=1 | 95 | AB E |
| 8 | LPEN EPGSS IMPG KVNP TQC | 35 8 | 37 7 | sp\|P07954\| FUMH_H UMAN Fumarate hydratase, mitochond rial OS=Homo sapiens OX=9606 GN=FH PE=1 SV=3 | FPEN EPGS SIMP GKV NPT QC | 30 8 | 32 7 | sp\|Q9Z6P6\|F UMC_CHLP N Fumarate hydratase class II OS=Chlamydi a pneumoniae OX=83558 GN=fumC PE=3 SV=1 | 95 | AB E |
| 9 | TSAL DTES EAEV Q | 56 0 | 57 2 | sp\|P21439\| MDR3_H UMAN Phosphatid ylcholine translocate r ABCB4 OS=Homo sapiens OX=9606 GN=ABC B4 PE=1 SV=2 | TSA LDT ESER A | 50 7 | 51 7 | tr\|B4ET30\|B4 ET30 PROM H ATP-dependent lipid A-core flippase OS=Proteus mirabilis (strain HI4320) OX=529507 GN=msbA PE=3 SV=1 | 91 | AB E |
| 10 | TSAL DTES EAEV Q | 56 0 | 57 2 | sp\|P21439\| MDR3_H UMAN Phosphatid ylcholine translocate r ABCB4 OS=Homo sapiens OX=9606 GN=ABC B4 PE=1 SV=2 | TSA LDT ESER A | 50 8 | 51 8 | tr\|A6T706\|A6 T706 KLEP7 ATP-dependent lipid A-core flippase OS=Klebsiell a pneumoniae subsp. pneumoniae (strain ATCC 700721 / MGH 78578) OX=272620 GN=msbA PE=3 SV=1 | 91 | AB E |
| 11 | TSAL DTES EAEV Q | 56 0 | 57 2 | sp\|P21439\| MDR3_H UMAN Phosphatid ylcholine translocate r ABCB4 OS=Homo sapiens OX=9606 GN=ABC B4 PE=1 SV=2 | TSA LDT ESER A | 50 8 | 51 8 | sp\|P60752\|MS BA_ECOLI ATP-dependent lipid A-core flippase OS=Escherich ia coli (strain K12) OX=83333 GN=msbA PE=1 SV=1 | 91 | AB E |
| 12 | IVIAH RLST | 58 5 | 59 4 | >sp\|P2143 9\|MDR3_ HUMAN Phosphatid ylcholine translocate r ABCB4 OS=Homo sapiens OX=9606 GN=ABC B4 PE=1 SV=2 | IVIA HRL ST | 52 5 | 53 4 | >tr\|A0A7Z3E 450\|A0A7Z3 E450_ACIBT Lipid A export permease/AT P-binding protein MsbA OS=Acinetob acter baumannii (strain ATCC 17978 / CIP 53.77 / LMG 1025 / NCDC KC755 / 5377) OX=400667 GN=msbA PE=4 SV=1 | 100 | MH CII |
| 13 | IVIAH RLST | 12 71 | 12 80 | >sp\|O9534 2\|ABCBB HUMAN Bile salt export pump OS=Homo sapiens OX=9606 GN=ABC B11 PE=1 SV=2 | IVIA HRL ST | 52 5 | 53 4 | >tr\|A0A7Z3E 450\|A0A7Z3 E450 ACIBT Lipid A export permease/AT P-binding protein MsbA OS=Acinetob acter baumannii (strain ATCC 17978 / CIP 53.77 / LMG 1025 / NCDC KC755 / 5377) OX=400667 GN=msbA PE=4 SV=1 | 100 | MH CII |
| 14 | VIAH RLSTI | 58 6 | 59 5 | >sp\|P2143 9\|MDR3_ HUMAN Phosphatid ylcholine translocate r ABCB4 OS=Homo sapiens OX=9606 GN=ABC B4 PE=1 SV=2 | VIA HRL STI | 52 6 | 53 5 | >tr\|A0A7Z3E 450\|A0A7Z3 E450_ACIBT Lipid A export permease/AT P-binding protein MsbA OS=Acinetob acter baumannii (strain ATCC 17978 / CIP 53.77 / LMG 1025 / NCDC KC755 / 5377) OX=400667 GN=msbA PE=4 SV=1 | 100 | MH CII |
| 15 | VIAH RLSTI | 12 72 | 12 81 | >sp\|O9534 2\|ABCBB HUMAN Bile salt export pump OS=Homo sapiens OX=9606 GN=ABC B11 PE=1 SV=2 | VIA HRL STI | 52 6 | 53 5 | >tr\|A0A7Z3E 450\|A0A7Z3 E450 ACIBT Lipid A export permease/AT P-binding protein MsbA OS=Acinetob acter baumannii (strain ATCC 17978 / CIP 53.77 / LMG 1025 / NCDC KC755 / 5377) OX=400667 GN=msbA PE=4 SV=1 | 100 | MH CII |
| 16 | LMLV TALN P | 45 3 | 46 1 | >sp\|P0795 4\|FUMH_ HUMAN Fumarate hydratase, mitochond rial OS=Homo sapiens OX=9606 GN=FH PE=1 SV=3 | LML VTA LNP | 40 5 | 41 4 | >tr\|A0A7Z3E 5C1\|A0A7Z3 E5C1_ACIBT Class II fumarate hydratase OS=Acinetob acter baumannii (strain ATCC 17978 / CIP 53.77 / LMG 1025 / NCDC KC755 / 5377) OX=400667 GN=fumC PE=4 SV=1 | 100 | MH CII |
| 17 | IVIAH RLST | 58 5,1 23 4 | 59 4, 12 43 | >sp\|P2143 9\|MDR3_ HUMAN Phosphatid ylcholine translocate r ABCB4 OS=Homo sapiens OX=9606 GN=ABC B4 PE=1 SV=2 | IVIA HRL ST | 53 4 | 54 3 | >tr\|B4EU63\|B 4EU63 PRO MH Putative efflux ABC transporter, ATP-binding membrane protein OS=Proteus mirabilis (strain HI4320) OX=529507 GN=mdlB PE=4 SV=1 | 100 | MH CII |
| 18 | IVIAH RLST | 12 71 | 12 81 | >sp\|O9534 2\|ABCBB HUMAN Bile salt export pump OS=Homo sapiens OX=9606 GN=ABC B11 PE=1 SV=2 | IVIA HRL ST | 53 4 | 54 3 | >tr\|B4EU63\|B 4EU63 PRO MH Putative efflux ABC transporter, ATP-binding membrane protein OS=Proteus mirabilis (strain HI4320) OX=529507 GN=mdlB PE=4 SV=1 | 100 | MH CII |
| 19 | VIAH RLSTI | 58 6,1 23 5 | 59 5, 12 44 | >sp\|P2143 9\|MDR3_ HUMAN Phosphatid ylcholine translocate r ABCB4 OS=Homo sapiens OX=9606 GN=ABC B4 PE=1 SV=2 | VIA HRL STI | 53 3 | 54 2 | >tr\|B4ET30\|B 4ET30 PRO MH ATP-dependent lipid A-core flippase OS=Proteus mirabilis (strain HI4320) OX=529507 GN=msbA PE=3 SV=1 | 100 | MH CII |
| 20 | VIAH RLSTI | 58 6,1 23 5 | 59 5, 12 44 | >sp\|P2143 9\|MDR3_ HUMAN Phosphatid ylcholine translocate r ABCB4 OS=Homo sapiens OX=9606 GN=ABC B4 PE=1 SV=2 | VIA HRL STI | 53 4 | 54 3 | >tr\|B4EU63\|B 4EU63 PRO MH Putative efflux ABC transporter, ATP-binding membrane protein OS=Proteus mirabilis (strain HI4320) OX=529507 GN=mdlB PE=4 SV=1 | 100 | MH CII |
| 21 | VIAH RLSTI | 12 72 | 12 80 | >sp\|O9534 2\|ABCBB HUMAN Bile salt export pump OS=Homo sapiens OX=9606 GN=ABC B11 PE=1 SV=2 | VIA HRL STI | 53 3 | 54 2 | >tr\|B4ET30\|B 4ET30 PRO MH ATP-dependent lipid A-core flippase OS=Proteus mirabilis (strain HI4320) OX=529507 GN=msbA PE=3 SV=1 | 100 | MH CII |
| 22 | VIAH RLSTI | 12 72 | 12 80 | >sp\|O9534 2\|ABCBB _HUMAN Bile salt export pump OS=Homo sapiens OX=9606 GN=ABC B11 PE=1 SV=2 | VIA HRL STI | 53 4 | 54 3 | >tr\|B4EU63\|B 4EU63 PRO MH Putative efflux ABC transporter, ATP-binding membrane protein OS=Proteus mirabilis (strain HI4320) OX=529507 GN=mdlB PE=4 SV=1 | 100 | MH CII |
| 23 | IVIAH RLST | 58 5,1 23 4 | 59 4, 12 43 | >sp\|P2143 9\|MDR3_ HUMAN Phosphatid ylcholine translocate r ABCB4 OS=Homo sapiens OX=9606 GN=ABC B4 PE=1 SV=2 | IVIA HRL ST | 52 7 | 53 6 | >tr\|Q2FVJ1\|Q 2FVJ1_STAA 8 Uncharacteriz ed protein OS=Staphyloc occus aureus (strain NCTC 8325 / PS 47) OX=93061 GN=SAOUH SC_02719 PE=4 SV=1 | 100 | MH CII |
| 24 | IVIAH RLST | 58 5,1 23 4 | 59 4, 12 43 | >sp\|P2143 9\|MDR3_ HUMAN Phosphatid ylcholine translocate r ABCB4 OS=Homo sapiens OX=9606 GN=ABC B4 PE=1 SV=2 | IVIA HRL ST | 52 5 | 53 4 | >tr\|Q2G2E5\| Q2G2E5_ST AA8 Uncharacteriz ed protein OS=Staphyloc occus aureus (strain NCTC 8325 / PS 47) OX=93061 GN=SAOUH SC_00647 PE=4 SV=1 | 100 | MH CII |
| 25 | IVIAH RLST | 12 71 | 12 80 | >sp\|O9534 2\|ABCBB _HUMAN Bile salt export pump OS=Homo sapiens OX=9606 GN=ABC B11 PE=1 SV=2 | IVIA HRL ST | 52 7 | 53 6 | >tr\|Q2FVJ1\|Q 2FVJ1_STAA 8 Uncharacteriz ed protein OS=Staphyloc occus aureus (strain NCTC 8325 / PS 47) OX=93061 GN=SAOUH SC_02719 PE=4 SV=1 | 100 | MH CII |
| 26 | IVIAH RLST | 12 71 | 12 80 | >sp\|O9534 2\|ABCBB HUMAN Bile salt export pump OS=Homo sapiens OX=9606 GN=ABC B11 PE=1 SV=2 | IVIA HRL ST | 52 5 | 53 4 | >tr\|Q2G2E5\| Q2G2E5_ST AA8 Uncharacteriz ed protein OS=Staphyloc occus aureus (strain NCTC 8325 / PS 47) OX=93061 GN=SAOUH SC_00647 PE=4 SV=1 | 100 | MH CII |
| 27 | VIAH RLSTI | 58 6,1 23 5 | 59 5, 12 44 | >sp\|P2143 9\|MDR3_ HUMAN Phosphatid ylcholine translocate r ABCB4 OS=Homo sapiens OX=9606 GN=ABC B4 PE=1 SV=2 | VIA HRL STI | 52 8 | 53 7 | >tr\|Q2FVJ1\|Q 2FVJ1_STAA 8 Uncharacteriz ed protein OS=Staphyloc occus aureus (strain NCTC 8325 / PS 47) OX=93061 GN=SAOUH SC_02719 PE=4 SV=1 | 100 | MH CII |
| 28 | VIAH RLSTI | 58 6,1 23 5 | 59 5, 12 44 | >sp\|P2143 9\|MDR3_ HUMAN Phosphatid ylcholine translocate r ABCB4 OS=Homo sapiens OX=9606 GN=ABC B4 PE=1 SV=2 | VIA HRL STI | 52 6 | 53 5 | >tr\|Q2G2E5\| Q2G2E5_ST AA8 Uncharacteriz ed protein OS=Staphyloc occus aureus (strain NCTC 8325 / PS 47) OX=93061 GN=SAOUH SC_00647 PE=4 SV=1 | 100 | MH CII |
| 29 | VIAH RLSTI | 12 72 | 12 81 | >sp\|O9534 2\|ABCBB HUMAN Bile salt export pump OS=Homo sapiens OX=9606 GN=ABC B11 PE=1 SV=2 | VIA HRL STI | 52 8 | 53 7 | >tr\|Q2FVJ1\|Q 2FVJ1_STAA 8 Uncharacteriz ed protein OS=Staphyloc occus aureus (strain NCTC 8325 / PS 47) OX=93061 GN=SAOUH SC_02719 PE=4 SV=1 | 100 | MH CII |
| 30 | VIAH RLSTI | 12 72 | 12 81 | >sp\|O9534 2\|ABCBB HUMAN Bile salt export pump OS=Homo sapiens OX=9606 GN=ABC B11 PE=1 SV=2 | VIA HRL STI | 52 6 | 53 5 | >tr\|Q2G2E5\| Q2G2E5_ST AA8 Uncharacteriz ed protein OS=Staphyloc occus aureus (strain NCTC 8325 / PS 47) OX=93061 GN=SAOUH SC_00647 PE=4 SV=1 | 100 | MH CII |
| 31 | LMLV TALN P | 45 3 | 46 1 | >sp\|P0795 4\|FUMH_ HUMAN Fumarate hydratase, mitochond rial OS=Homo sapiens OX=9606 GN=FH PE=1 SV=3 | LML VTA LNP | 40 4 | 41 3 | >tr\|Q2FX94\|Q 2FX94_STA A8 Fumarate hydratase class II OS=Staphyloc occus aureus (strain NCTC 8325 / PS 47) OX=93061 GN=fumC PE=3 SV=1 | 100 | MH CII |
| 32 | TIVIA HRLS | 58 4 | 59 3 | >sp\|P2143 9\|MDR3_ HUMAN Phosphatid ylcholine translocate r ABCB4 OS=Homo sapiens OX=9606 GN=ABC B4 PE=1 SV=2 | TIVI AHR LS | 52 4 | 53 3 | >tr\|Q2G2E5\| Q2G2E5_ST AA8 Uncharacteriz ed protein OS=Staphyloc occus aureus (strain NCTC 8325 / PS 47) OX=93061 GN=SAOUH SC_00647 PE=4 SV=1 | 100 | MH CII |
| 33 | RIAIA RALV | 54 0 | 54 9 | >sp\|P2143 9\|MDR3_ HUMAN Phosphatid ylcholine translocate r ABCB4 OS=Homo sapiens OX=9606 GN=ABC B4 PE=1 SV=2 | RIAI ARA LV | 15 0 | 15 9 | ^{>}tr\|Q2FUZ1\| Q2FUZ 1 ST AA8 ABC transporter, ATP-binding protein, putative OS=Staphyloc occus aureus (strain NCTC 8325 / PS 47) OX=93061 GN=SAOUH SC_02954 PE=4 SV=1 | 100 | MH CII |

### Atopic dermatitis (AD):

Atopic dermatitis (AD) is a chronic inflammation of the skin resulting in itchy, red, swollen, and cracked skin.it. The AD affects about 15-20% of children and is mostly characterized by involvement of both non-immune and immune components of the skin-barrier Dys functioning. The cause is unknown but various studies associate genetics, immune system dysfunction, environmental exposures, and difficulties with the permeability of the skin like factors to trigger the AD. Aberrant immune response against harmless allergens, loss in membrane integrity of skin by mutation in filaggrin (FLG) resulting in increased permeability and disintegration of skin membranes so as to expose internal components to the allergens/microbial agents which can cause infection are one of the proposed mechanisms of the trigger. Severe immune reaction to self-proteins is also observed in AD patients. Clinical manifestations are increased IgE levels showing strong association of AD with IgE-autoreactivity as well as with ANA (anti-nuclear antibodies) and showing sensitization to various allergens including e.g., aero, food, and microbial allergens. Microbial dysbiosis in the skin and gut are associated with the severity of AD. The inflammatory response to fungi, especially Malassezia, is an important trigger in AD. Malassezia restricta and *Malassezia globosa* are the two major subtypes isolated from the skin samples of patients in AD. AD associated dysbiosis in microbiota is well characterized by the colonization by Staphylococcus aureus, with decrease in the abundance of other potentially beneficial species. S. aureus colonizes skin by anchoring and making biofilms, and expressing several virulence factors leading to the pathogenesis in AD.

In certain studies, molecular mimicry is proposed to be one of the mechanisms by which microbial interaction with the host results in the generation of autoreactive IgE antibodies. For example, in one of the study roles of stress-inducible enzyme manganese superoxide dismutase (MnSOD) of human as an auto allergen in AD showing significant molecular mimicry to fungal dismutase was investigated. Cross reactive sensitization was observed in AD patients as a result of exposure to environmental fungal MnSOD of Malassezia sympodialis. Similarly, cross reactive IgEs are also observed against the epitopes from thioredoxins (Trx) of human and fungal origin showing molecular similarity leading to allergic responses. Treatment options is mostly limited to anti-inflammatory agents that broadly suppress inflammation and have poor efficacy, safety and/or tolerability. In the present disclosure, the molecular mimicry mechanism involving microbial host interaction leading to increased autoimmune response resulting in disease exacerbation, are exploited. The major microbial species from Staphylococcus aureus MW2, Staphylococcus aureus MRSA252, Staphylococcus haemolyticus (strain JCSC1435, Malassezia restricta CBS 7877, Staphylococcus aureus NCTC 8325 implicated in the AD severity are taken for analysis. The list of RMME specific to the AD condition were identified using the sample steps as described in the methodology.

In one embodiment the listed sequence IDs D1_1 to D1_35 in Table 5, can be used as immunomodulating composition capable of modulating the immune system in order to subside the symptoms of the AD. Further, D1_RMME_detail_map is created which comprises list of microbial epitopes implicated in AD and are specified in Table 7.

**Table 7**

| S. no | epito pe(hu man) | Sta rt | end | human protein info | epitope (microb e) | Sta rt | End | microbe protein info | matc h | Type |
|---|---|---|---|---|---|---|---|---|---|---|
| 1 | VRP SGT ED | 49 5 | 502 | sp\|O95 394\|AG M1_H UMAN Phosph oacetyl glucosamine mutase OS=Ho mo sapiens OX=96 06 GN=P GM3 PE=1 SV=1 | VQKT PQGRS FVRPS GTED | 50 2 | 519 | tr\|A0A3G 2S1K2\|A0 A3G2S1K 2_9BASI Phosphoac etylglucos amine mutase OS=Malas sezia restricta CBS 7877 OX=4252 64 GN=DNF 11_0894 PE=3 SV=1 | 100 | ABE |
| 2 | DWN PQN DLQ A | 12 30 | 123 9 | sp\|Q9H CK8\|C HD8_H UMAN Chrom odomai n-helicas e-DNA-binding protein 8 OS=Ho mo sapiens OX=9606 GN=C HD8 PE=1 SV=5 | DWNP QNDL Q | 73 2 | 740 | tr\|A0A3G 2S0Q8\|A0 A3G2S0Q 8_9BASI Chromodo main helicase hrp3 OS=Malas sezia restricta CBS 7877 OX=4252 64 GN=hrp3 PE=4 SV=1 | 100 | ABE |
| 3 | PGH VDF SSE | 94 | 102 | sp\|Q7Z 2Z2\|EF L1_HU MAN Elongat ion factor-like GTPase 1 OS=Ho mo sapiens OX=96 06 GN=EF L1 PE=1 SV=2 | PGHV DFSSE VS | 97 | 107 | tr\|A0A3G 2SB10\|A0 A3G2SB1 0_9BASI Ribosome assembly protein 1 OS=Malas sezia restricta CBS 7877 OX=4252 64 GN=ria1 PE=4 SV=1 | 100 | ABE |
| 4 | YPQ YSC STT GS | 19 1 | 201 | sp\|P228 30\|HE MH_H UMAN | YPQY SCSTT GSS | 11 9 | 130 | tr\|A0A3G 2S4V4\|A0 A3G2S4V 4_9BASI | 100 | ABE |
| | | | | Ferroch elatase, mitoch ondrial | | | | Ferrochela tase, mitochond rial | | |
| | | | | OS=Ho mo sapiens | | | | OS=Malas sezia restricta | | |
| | | | | OX=96 06 | | | | CBS 7877 | | |
| | | | | | | | | OX=4252 64 | | |
| | | | | GN=FE | | | | | | |
| | | | | CH | | | | GN=fech | | |
| | | | | PE=1 | | | | PE=3 | | |
| | | | | SV=2 | | | | SV=1 | | |
| 5 | ITPV PGG VGP | 26 8 | 277 | sp\|P115 | ITPVP GGVG P | 27 4 | 283 | tr\|A0A3G | 100 | ABE |
| | | | | 86\|C1T | | | | 2S8A1\|A0 | | |
| | | | | C_HU | | | | A3G2S8A | | |
| | | | | MAN | | | | 1_9BASI | | |
| | | | | C-1-tetrahy drofolat e synthas e, cytopla smic | | | | Formyltetr ahydrofola te synthetase OS=Malas sezia restricta | | |
| | | | | | | | | CBS 7877 | | |
| | | | | OS=Ho mo sapiens OX=96 06 GN=M THFD1 PE=1 SV=4 | | | | OX=4252 64 | | |
| | | | | | | | | GN=DNF 11_3365 PE=3 SV=1 | | |
| 6 | ITPT PLG EGK STT | 37 7 | 389 | sp\|P115 86\|C1T C_HU MAN C-1-tetrahy drofolat e synthas e, cytopla smic OS=Ho mo sapiens OX=96 06 GN=M THFD1 PE=1 SV=4 | ITPTP LGEG KSTTT | 38 4 | 397 | tr\|A0A3G 2S8A1\|A0 A3G2S8A 1_9BASI Formyltetr ahydrofola te synthetase OS=Malas sezia restricta CBS 7877 OX=4252 64 GN=DNF 11_3365 PE=3 SV=1 | 100 | ABE |
| 7 | QPS QGP TFGI KGG AAG GGY SQV | 41 1 | 432 | sp\|P115 86\|C1T C_HU MAN C-1-tetrahy drofolat e synthas e, cytopla smic OS=Ho mo sapiens OX=96 06 GN=M THFD1 PE=1 SV=4 | RQPSQ GPTFG IKGGA AGGG YSQV | 41 7 | 439 | tr\|A0A3G 2S8A1\|A0 A3G2S8A 1_9BASI Formyltetr ahydrofola te synthetase OS=Malas sezia restricta CBS 7877 OX=4252 64 GN=DNF 11_3365 PE=3 SV=1 | 100 | ABE |
| 8 | WDY QPDI CKD YKE T | 19 5 | 208 | sp\|O15 541\|R1 13A_H UMAN E3 ubiquiti n-protein ligase RNF11 3A OS=Ho mo sapiens OX=96 06 GN=R NF113 APE=1 SV=1 | PDICK DYKE T | 15 4 | 163 | tr\|A0A3G 2S147\|A0 A3G2S14 7_9BASI Pre-mRNA-splicing factor CWC24 OS=Malas sezia restricta CBS 7877 OX=4252 64 GN=CWC 24 PE=3 SV=1 | 100 | ABE |
| 9 | DWN PHQ DLQ AQD RAH RIGQ Q | 11 47 | 116 6 | sp\|P515 31\|SM CA2_H UMAN Probabl e global transcri ption activato r SNF2L 2 OS=Ho mo sapiens OX=96 06 GN=S MARC A2 PE=1 SV=2 | DWNP HQDL QAQD RAHR | 96 5 | 980 | tr\|A0A3G 2S1A4\|A0 A3G2S1A 4_9BASI Chromatin structure-remodelin g complex subunit snf21 OS=Malas sezia restricta CBS 7877 OX=425264 GN=snf21 PE=4 SV=1 | 100 | ABE |
| 10 | HGG SRR QEA Q | 62 9 | 638 | sp\|P194 47\|ERC C3_HU MAN General transcri ption and DNA repair factor IIH helicas e subunit XPB OS=Ho mo sapiens OX=96 06 GN=E RCC3 PE=1 SV=1 | GSRR QEAQ | 56 7 | 574 | tr\|A0A3G 2SDA2\|A 0A3G2SD A2_9BAS I DNA helicase OS=Malas sezia restricta CBS 7877 OX=4252 64 GN=ercc3 PE=3 SV=1 | 100 | ABE |
| 11 | TAG QED YDR L | 58 | 67 | sp\|P630 00\|RA C1_HU MAN Ras-related C3 botulin um toxin substrat e 1 OS=Ho mo sapiens OX=96 06 GN=R AC1 PE=1 SV=1 | TAGQ EDYD RL | 58 | 67 | tr\|A0A3G 2S438\|A0 A3G2S43 8_9BASI Ras-related C3 botulinum toxin substrate 1 OS=Malas sezia restricta CBS 7877 OX=4252 64 GN=Rac 1 PE=4 SV=1 | 100 | ABE |
| 12 | TAG QED | 58 | 67 | sp\|P630 00\|RA C1_HU | | 69 | 78 | tr\|A0A3G 2S7A1\|A0 A3G2S7A | 100 | ABE |
| | YDR L | | | MAN Ras-related C3 botulin um toxin substrat e 1 OS=Ho mo sapiens OX=96 06 GN=R AC1 PE=1 SV=1 | | | | 1_9BASI GTP-binding protein rho1 OS=Malas sezia restricta CBS 7877 OX=4252 64 GN=rho 1 PE=4 SV=1 | | |
| 13 | TAG QED YDR L | 58 | 67 | sp\|P630 00\|RA C1_HU MAN Ras-related C3 botulin um toxin substrat e 1 OS=Ho mo sapiens OX=96 06 GN=R AC1 PE=1 SV=1 | TAGQ EDYD RL | 58 | 67 | tr\|A0A3G 2SA52\|A0 A3G2SA5 2_9BASI Cell division control protein 42 homolog OS=Malas sezia restricta CBS 7877 OX=4252 64 GN=CDC 42 PE=3 SV=1 | 100 | ABE |
| 14 | EMP SGT GK | 41 | 48 | sp\|P180 74\|ERC C2_HU MAN General transcri ption and DNA repair factor IIH helicas e subunit XPD OS=Homo sapiens OX=96 06 GN=E RCC2 PE=1 SV=1 | EMPS GTGK | 41 | 48 | tr\|A0A3G 2S3K2\|A0 A3G2S3K 2_9BASI General transcripti on and DNA repair factor IIH helicase subunit XPD OS=Malas sezia restricta CBS 7877 OX=4252 64 GN=rad15 PE=3 SV=1 | 100 | ABE |
| 15 | QWG DEG KGK | 37 | 45 | sp\|P305 20\|PUR A2_HU MAN Adenyl osuccin ate synthet ase isozym e 2 OS=Ho mo sapiens OX=96 06 GN=A DSS2 PE=1 SV=3 | QWGD EGKG K | 22 | 30 | tr\|A0A3G 2S1Z0\|A0 A3G2S1Z 0_9BASI Adenylosu ccinate synthetase OS=Malas sezia restricta CBS 7877 OX=4252 64 GN=DNF 11_0836 PE=3 SV=1 | 100 | ABE |
| 16 | EPTT NLD RE | 12 32 | 124 0 | sp\|Q92 878\|RA D50_H UMAN DNA repair protein RAD50 OS=Ho mo sapiens OX=96 06 GN=R AD50 PE=1 SV=1 | DEPTT NLDR E | 97 4 | 983 | tr\|A0A3G 2S370\|A0 A3G2S37 0_9BASI DNA repair protein RAD50 OS=Malas sezia restricta CBS 7877 OX=4252 64 GN=RAD 50 PE=4 SV=1 | 100 | ABE |
| 17 | TLSE ESY K | 20 5 | 212 | sp\|P631 04\|1433 Z_HU MAN 14-3-3 protein zeta/del ta OS=Ho mo sapiens OX=96 06 GN=Y WHAZ PE=1 SV=1 | TLSEE SYK | 21 0 | 217 | tr\|A0A3G 2SAK1\|A 0A3G2SA K1_9BAS I DNA damage checkpoin t protein rad24 OS=Malas sezia restricta CBS 7877 OX=4252 64 GN=rad24 PE=3 SV=1 | 100 | ABE |
| 18 | SGA GNN WAK GHY TEG AE | 95 | 111 | sp\|P683 71\|TBB 4B_HU MAN Tubulin beta-4B chain OS=Ho mo sapiens OX=96 06 GN=T UBB4 B PE=1 SV=1 | SGAG NNWA KGHY TEGA E | 95 | 111 | tr\|A0A3G 2S9J3\|A0 A3G2S9J 3_9BASI Tubulin beta chain OS=Malas sezia restricta CBS 7877 OX=4252 64 GN=TUB 1 PE=3 SV=1 | 100 | ABE |
| 19 | GEG MDE MEF TEA | 40 0 | 415 | sp\|P683 71\|TBB 4B_HU MAN Tubulin | GEGM DEME FTEAE S | 40 0 | 413 | tr\|A0A3G 2S9J3\|A0 A3G2S9J 3_9BASI Tubulin | 100 | ABE |
| | ESN M | | | beta-4B chain OS=Ho mo sapiens OX=96 06 GN=T UBB4 B PE=1 SV=1 | | | | beta chain OS=Malas sezia restricta CBS 7877 OX=4252 64 GN=TUB 1 PE=3 SV=1 | | |
| 20 | ANE AKG TKV V | 44 | 53 | sp\|P522 09\|6PG D_HU MAN 6-phosph ogluco nate dehydr ogenas e, decarbo xylatin g OS=Ho mo sapiens OX=96 06 GN=P GD PE=1 SV=3 | ANEA KGTK VV | 47 | 56 | tr\|A0A3G 2S6E8\|A0 A3G2S6E 8_9BASI 6-phosphogl uconate dehydroge nase, decarboxy lating OS=Malas sezia restricta CBS 7877 OX=4252 64 GN=GND 2 PE=3 SV=1 | 100 | ABE |
| 21 | RDS AGQ KGT GKW T | 25 5 | 267 | sp\|P522 09\|6PG D_HU MAN 6-phosph ogluco nate dehydr ogenas e, decarbo xylatin g OS=Ho mo sapiens OX=96 06 GN=P GD PE=1 SV=3 | DSAG QKGT GKWT | 25 8 | 269 | tr\|A0A3G 2S6E8\|A0 A3G2S6E 8_9BASI 6-phosphogl uconate dehydroge nase, decarboxy lating OS=Malas sezia restricta CBS 7877 OX=4252 64 GN=GND 2 PE=3 SV=1 | 100 | ABE |
| 22 | TELP PTHP | 16 1 | 168 | sp\|P622 5811433 E_HU MAN 14-3-3 protein epsilon OS=Ho mo sapiens OX=96 06 GN=Y WHAE PE=1 SV=1 | ITELP PTHP | 16 2 | 170 | tr\|A0A3G 2SAK1\|A 0A3G2SA K1_9BAS I DNA damage checkpoin t protein rad24 OS=Malas sezia restricta CBS 7877 OX=4252 64 GN=rad24 PE=3 SV=1 | 100 | ABE |
| 23 | TLSE ESY K | 20 8 | 215 | sp\|P622 5811433 E_HU MAN 14-3-3 protein epsilon OS=Homo sapiens OX=96 06 GN=Y WHAE PE=1 SV=1 | TLSEE SYK | 21 0 | 217 | tr\|A0A3G 2SAK1\|A 0A3G2SA K1_9BAS I DNA damage checkpoin t protein rad24 OS=Malas sezia restricta CBS 7877 OX=4252 64 GN=rad24 PE=3 SV=1 | 100 | ABE |
| 24 | VPS GAS TG | 35 | 42 | sp\|P067 33\|EN OA_H UMAN Alpha-enolase OS=Ho mo sapiens OX=96 06 GN=E NO1 PE=1 SV=2 | VPSG ASTG VH | 65 | 74 | tr\|A0A3G 2SD22\|A0 A3G2SD2 2_9BASI Phosphop yruvate hydratase OS=Malas sezia restricta CBS 7877 OX=4252 64 GN=DNF 11_3717 PE=3 SV=1 | 100 | ABE |
| 25 | RSG ETE DT | 37 2 | 379 | sp\|P067 33\|EN OA_H UMAN Alpha-enolase OS=Ho mo sapiens OX=96 06 GN=E NO1 PE=1 SV=2 | RSGET EDTTI | 40 3 | 412 | tr\|A0A3G 2SD22\|A0 A3G2SD2 2_9BASI Phosphop yruvate hydratase OS=Malas sezia restricta CBS 7877 OX=4252 64 GN=DNF 11_3717 PE=3 SV=1 | 100 | ABE |
| 26 | ITPV PGG VGP | 26 8 | 277 | sp\|P115 86\|C1T C_HU MAN C-1-tetrahy drofolat e synthas e, cytoplasmic OS=Ho mo sapiens OX=96 06 GN=M THFD1 PE=1 SV=4 | GAITP VPGG VGP | 25 3 | 264 | sp\|Q2FZJ 6\|FOLD_ STAA8 Bifunction al protein FolD OS=Staph ylococcus aureus (strain NCTC 8325 / PS 47) OX=9306 1 GN=folD PE=3 SV=1 | 100 | ABE |
| 27 | QWG DEG KGK | 37 | 45 | sp\|P305 20\|PUR A2_HU MAN Adenyl osuccin ate synthet ase isozym e 2 OS=Ho mo sapiens OX=96 06 GN=A DSS2 PE=1 SV=3 | QWGD EGKG K | 10 | 18 | tr\|Q2G1S3 \|Q2G1S3_ STAA8 Adenylosu ccinate synthetase OS=Staph ylococcus aureus (strain NCTC 8325 / PS 47) OX=9306 1 GN=purA PE=3 SV=1 | 100 | ABE |
| 28 | DSR GNP TV | 13 | 20 | sp\|P067 33\|EN OA_H UMAN Alpha-enolase OS=Ho mo sapiens OX=96 06 GN=E NO1 PE=1 SV=2 | LDSR GNPT V | 13 | 21 | sp\|Q2G02 8\|ENO_S TAA8 Enolase OS=Staph ylococcus aureus (strain NCTC 8325 / PS 47) OX=9306 1 GN=eno PE=1 SV=1 | 100 | ABE |
| 29 | VPS GAS TG | 35 | 42 | sp\|P067 33\|EN OA_H UMAN Alpha-enolase OS=Ho mo sapiens OX=96 06 GN=E NO1 PE=1 SV=2 | VPSG ASTGE HEAV ELRD GDKS RY | 37 | 59 | sp\|Q2G02 8\|ENO_S TAA8 Enolase OS=Staph ylococcus aureus (strain NCTC 8325 / PS 47) OX=9306 1 GN=eno PE=1 SV=1 | 100 | ABE |
| 30 | RSG ETE DT | 37 2 | 379 | sp\|P067 33\|EN OA_H UMAN Alpha-enolase OS=Ho mo sapiens OX=96 06 GN=E NO1 PE=1 SV=2 | HRSG ETEDT TI | 37 1 | 381 | sp\|Q2G02 8\|ENO_S TAA8 Enolase OS=Staph ylococcus aureus (strain NCTC 8325 / PS 47) OX=9306 1 GN=eno PE=1 SV=1 | 100 | ABE |
| 31 | VGP SGA GK | 62 2 | 629 | sp\|Q9N P58\|AB CB6_H UMAN ATP-binding cassette subfamily B membe r 6 OS=Ho mo sapiens OX=96 06 GN=A BCB6 PE=1 SV=1 | LVGPS GAGK S | 35 5 | 364 | tr\|Q2G0B 3\|Q2G0B3 STAA8 Uncharact erized protein OS=Staph ylococcus aureus (strain NCTC 8325 / PS 47) OX=9306 1 GN=SAO UHSC_00 692 PE=4 SV=1 | 100 | ABE |
| 32 | ITPV PGG VGP | 26 8 | 277 | sp\|P115 86\|C1T C_HU MAN C-1-tetrahy drofolat e synthas e, cytopla smic OS=Ho mo sapiens OX=9606 GN=M THFD1 PE=1 SV=4 | GAITP VPGG VGP | 25 3 | 264 | sp\|Q6GI2 1\|FOLD_ STAAR Bifunction al protein FolD OS=Staph ylococcus aureus (strain MRSA252 ) OX=2824 58 GN=folDPE=3 SV=1 | 100 | ABE |
| 33 | QWG DEG KGK | 37 | 45 | sp\|P305 20\|PUR A2_HU MAN Adenyl osuccin ate synthet ase isozym e 2 OS=Ho mo sapiens OX=96 06 GN=A DSS2 PE=1 SV=3 | QWGD EGKG K | 10 | 18 | sp\|Q6GKS 8\|PURA_ STAAR Adenylosu ccinate synthetase OS=Staph ylococcus aureus (strain MRSA252) ) OX=2824 58 GN=purA PE=3 SV=1 | 100 | ABE |
| 34 | DSR GNP TV | 13 | 20 | sp\|P067 33\|EN OA_HUMAN Alpha-enolase OS=Ho mo sapiens OX=96 06 GN=E NO1 PE=1 SV=2 | | 13 | 21 | sp\|Q6GIL 4\|ENO_S TAAR Enolase OS=Staph ylococcus aureus (strain MRSA252 ) OX=2824 58 GN=eno PE=3 SV=1 | 100 | ABE |
| 35 | VPS GAS TG | 35 | 42 | sp\|P067 33\|EN OA_H UMAN Alpha-enolase OS=Ho mo sapiens OX=96 06 GN=E NO1 PE=1 SV=2 | VPSG ASTGE HEAV ELRD GDKS RY | 37 | 59 | sp\|Q6GIL 4\|ENO_S TAAR Enolase OS=Staph ylococcus aureus (strain MRSA252) ) OX=2824 58 GN=eno PE=3 SV=1 | 100 | ABE |
| 36 | RSG ETE DT | 37 2 | 379 | sp\|P067 33\|EN OA_H UMAN Alpha-enolase OS=Ho mo sapiens OX=96 06 GN=E NO1 PE=1 SV=2 | HRSG ETEDT TI | 37 1 | 381 | sp\|Q6GIL 4\|ENO_S TAAR Enolase OS=Staph ylococcus aureus (strain MRSA252 ) OX=2824 58 GN=eno PE=3 SV=1 | 100 | ABE |
| 37 | VGP SGA GK | 62 2 | 629 | sp\|Q9N P58\|AB CB6_H UMAN ATP-binding cassette subfamily B membe r 6 OS=Homo sapiens OX=96 06 GN=A BCB6 PE=1 SV=1 | LVGPS GAGK S | 35 5 | 364 | tr\|A0A7U 7EU95\|A0 A7U7EU9 5_STAAR ABC transporter ATP-binding protein OS=Staph ylococcus aureus (strain MRSA252 ) OX=2824 58 GN=SAR 0737 PE=4 SV=1 | 100 | ABE |
| 38 | ITPV PGG VGP | 26 8 | 277 | sp\|P115 86\|C1T C_HU MAN C-1-tetrahy drofolat e synthas e, cytopla smic OS=Ho mo sapiens OX=96 06 GN=M THFD1PE=1 SV=4 | GAITP VPGG VGP | 25 3 | 264 | sp\|Q4L57 2\|FOLD_ STAHJ Bifunction al protein FolD OS=Staph ylococcus haemolyti cus (strain JCSC1435) ) OX=2798 08 GN=folD PE=3 SV=1 | 100 | ABE |
| 39 | QWG DEG KGK | 37 | 45 | sp\|P305 20\|PUR A2_HU MAN Adenyl osuccin ate synthet ase isozym e 2 OS=Ho mo sapiens OX=96 06 GN=A DSS2 PE=1 SV=3 | QWGD EGKG K | 10 | 18 | sp\|Q4LA K0\|PURA _STAIJ Adenylosu ccinate synthetase OS=Staph ylococcus haemolyti cus (strain JCSC1435) ) OX=2798 08 GN=purA PE=3 SV=1 | 100 | ABE |
| 40 | DSR GNP TV | 13 | 20 | sp\|P067 33\|EN OA_H UMAN Alpha-enolase OS=Ho mo sapiens OX=96 06 GN=E NO1 PE=1 SV=2 | LDSR GNPT V | 13 | 21 | sp\|Q4L4K 7\|ENO_S TAHJ Enolase OS=Staph ylococcus haemolyti cus (strain JCSC1435 ) OX=2798 08 GN=eno PE=3 SV=1 | 100 | ABE |
| 41 | VPS GAS TG | 35 | 42 | sp\|P067 33\|EN OA_H UMAN Alpha-enolase OS=Ho mo sapiens OX=96 06 GN=E NO1 PE=1 SV=2 | VPSG ASTGE HEAV ELRD GDKS RYSG KGVT KAVE | 37 | 69 | sp\|Q4L4K 7\|ENO_S TAHJ Enolase OS=Staph ylococcus haemolyti cus (strain JCSC1435) ) OX=2798 08 GN=eno PE=3 SV=1 | 100 | ABE |
| 42 | RSG ETE DT | 37 2 | 379 | sp\|P067 33\|EN OA_H UMAN Alpha-enolase OS=Ho mo sapiens OX=96 06 GN=E NO1 PE=1 SV=2 | HRSG ETEDT TI | 37 1 | 381 | sp\|Q4L4K 7\|ENO_S TAHJ Enolase OS=Staph ylococcus haemolyti cus (strain JCSC1435) OX=2798 08 GN=eno PE=3 SV=1 | 100 | ABE |
| 43 | VGP SGA GK | 62 2 | 629 | sp\|Q9N P58\|AB CB6_H UMAN ATP-binding cassette subfamily B member 6 OS=Ho mo sapiens OX=96 06 GN=A BCB6 PE=1 SV=1 | NVAIV GPSG AGKT | 35 2 | 364 | tr\|Q4L4A 7\|Q4L4A7 STAID Uncharact erized protein OS=Staph ylococcus haemolyti cus (strain JCSC1435 ) OX=2798 08 GN=SH22 09 PE=4 SV=1 | 100 | ABE |
| 44 | KLE KER QEQ LQK EQE | 53 | 67 | sp\|Q16 236\|NF 2L2_H UMAN Nuclear factor erythroid 2-related factor 2 OS=Ho mo sapiens OX=96 06 GN=N FE2L2 PE=1 SV=3 | KLEK ERQE | 23 | 30 | tr\|Q4L4M 8\|Q4L4M 8_STAHJ Uncharact erized protein OS=Staph ylococcus haemolyti cus (strain JCSC1435) OX=2798 08 GN=SH20 88 PE=4 SV=1 | 100 | ABE |
| 45 | ATL SVH QLV | 23 1 | 239 | P68371 | ATLSV HQLV | 18 5 | 194 | Malassezi a_restricta CBS78 77, A0A3G2S9J3_9BAS I | 100 | MHC II |
| 46 | LRFL RYG SL | 21 1 | 220 | Q8N46 5 | LRFLR YGSL | 19 7 | 206 | *Malassezi a_restrict a_CBS_78* 77, A0A3G2S 699_9BA SI | 100 | MHC II |
| 47 | | 73 2 | 741 | Q9NP5 8 | QRVAI ARTI | 70 6 | 715 | *Malassezi a_restrict a_CBS_78* 77, A0A3G2S 3S0_9BA S | 100 | MHC II |
| 48 | RITL TSR NV | 21 | 29 | P60866 | RITLT SRNV | 22 | 31 | *Malassezi a_restrict a_CBS_78* 77, A0A3G2S 860_9BA SI | 100 | MHC II |
| 49 | SRG NPT VEV | 14 | 22 | P06733 | SRGNP TVEV | 15 | 24 | *Staphyloc occus aureus (strain MRSA252*), Q6GIL4\|E NO_STA AR | 100 | MHC II |

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the embodiments or if they include equivalent elements with insubstantial differences from the literal language of the embodiments described herein.

The embodiments of present disclosure herein address unresolved problem of treating and handling the autoimmune disorders effectively by identifying the microbial epitopes present in the sample of the subject and by mapping the identified epitopes through the epitope knowledgebase 116.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means, and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development changes the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated herein by the following claims.

## Claims

1. A method (200) comprising the steps of:
collecting a biological sample from a subject for whom one or more autoimmune disorders to be handled (202);
extracting one or more DNA sequences, from the biological sample, using one or more DNA sequence extraction techniques (204);
identifying microbial taxa from the one or more DNA sequences of the biological sample, using one or more microbial taxa extraction techniques, wherein the microbial taxa is composed of one or more microbes predominantly present in the biological sample (206);
identifying, via one or more hardware processors, one or more pathogenic microbes, by mapping pathogenic microbial taxa obtained from the microbial taxa to the pathogenic microbial taxa present in a disease microbe map (DM map) of an epitope knowledgebase (208);
identifying one or more microbial peptides from the biological sample, using one or more microbial peptides identification techniques (210);
identifying, via the one or more hardware processors, one or more microbial epitopes, from the one or more microbial peptides present in the biological sample, using the epitope knowledgebase (212);
assessing, via the one or more hardware processors, one or more autoimmune disorders of the subject, based on at least one of (i) the one or more pathogenic microbes and (ii) the one or more microbial epitopes, using the epitope knowledgebase (214); and
generating, via the one or more hardware processors, an artificial sequence construct, using one or more mimic epitopes identified from the epitope knowledgebase, wherein the artificial sequence construct comprises of one or more refined molecular mimic epitopes associated to the one or more autoimmune disorders present in the epitope knowledgebase linked by (i) one or more peptide linkers, (ii) one or more adjuvants, and (iii) one or more toll-like receptor (TLR) ligands, to enhance an immunogenicity (216).

2. The method as claimed in claim 1, wherein
the one or more refined molecular mimic epitopes associated to the one or more autoimmune disorders are utilized to generate one or more monoclonal antibodies for administering the subject to impede a disease progression;
the one or more peptide linkers are selected from a group consisting of: GGGS, AAY, KK, GPGPG, and HEYGAEALERAG, and wherein B cell epitopes and HTL epitopes (MHC II epitopes) can be linked by KK and GPGPG peptide linker;
the one or more adjuvants are selected from a group consisting of: particulate emulsions, microparticles, iscoms cochleates, and liposomes, and wherein the one or more adjuvants are fused using a EAAAK linker;
the one or more toll-like receptor (TLR) ligands are selected from a group consisting of: MPL (TLR2 and TLR4 ligands), CpG ODN (TLR9 ligand), CTB-CpG, Flagellin (TLR5 ligand); and
the one or more pathogenic microbes comprising the epitope identified in a disease condition is eliminated using antimicrobials, wherein the antimicrobials comprise a microbial modulation in the form of probiotics, prebiotics or the antimicrobials that target the one or more pathogenic microbes in the biological sample in order to control disease progression.

3. The method as claimed in claim 1, wherein
the artificial sequence construct comprises a combination of one or more epitope sequences from a sequence identifier (ID) D2_1 to D2_16 listed in Table 4, the one or more peptide linkers, the one or more adjuvants or the one or more toll-like receptor (TLR) ligands, for an autoimmune disorder being a primary biliary cholangitis (PBC); and
the artificial sequence construct comprises a combination of one or more epitope sequences from a sequence identifier (ID) D1_1 to D1_35 listed in Table 5, the one or more peptide linkers, the one or more adjuvants or the one or more toll-like receptor (TLR) ligands, for an autoimmune disorder being an Atopic Dermatitis (AD).

4. The method as claimed in claim 1, further comprising assessing an efficacy of the artificial sequence construct generated to the subject (218).

5. The method as claimed in claim 1, wherein the epitope knowledgebase comprises a DM_map, a X_RMME_map, and a X_RMME_detail_map, wherein the DM_map comprises mapping of the one or more autoimmune disorders to a set of microbes, the X_RMME_map comprises mapping of one or more refined molecular mimic epitopes to each of the one or more autoimmune disorders, and the X_RMME_detail_map comprises a detailed information of each of the one or more refined molecular mimic epitopes present in the X_RMME_map.

6. The method as claimed in claim 1, wherein
the one or more refined molecular mimic epitopes of an autoimmune disorder being a primary biliary cholangitis (PBC), are listed in Table 4 in the form of epitope sequences from a sequence identifier (ID) D2_1 to D2_16; and
the one or more refined molecular mimic epitopes of an autoimmune disorder being an Atopic Dermatitis (AD), are listed in Table 5 in the form of epitope sequences from a sequence identifier (ID) D1_1 to D1_35.

7. The method as claimed in claim 1, wherein the epitope knowledgebase is created by:
identifying a plurality of disease specific proteomes pertaining to the one or more autoimmune disorders, using one or more data mining techniques;
predicting one or more disease specific epitopes for each of the one or more autoimmune disorders, from the plurality of disease specific proteomes, based on a binding capability;
identifying one or more potential molecular mimic epitopes, for each of the one or more autoimmune disorders, from the one or more disease specific epitopes, that show a sequence similarity with self-peptides and results in cross-activation of autoreactive T; and
refining the one or more potential molecular mimic epitopes, to obtain one or more refined molecular mimic epitopes for each of the one or more autoimmune disorders, using one or more of (i) a structural superimposition and analysis, (ii) a cellular localization prediction, (iii) a proteosome processing, and (iv) an immunogenicity prediction.

8. A system (100) comprising:
one or more hardware processors 102;
a memory 104;
input/output (I/O) interfaces 106;
a sample collection module 108;
a DNA extraction and sequencing module 110;
a peptide identification module 112;
an epitope identification module 114;
an epitope knowledgebase 116;
a disease assessment module 118;
a sequence construct generation module 120;
an efficacy assessment module 122; and
wherein the one or more hardware processors 102 are configured by the instructions to perform one or more of:
collecting a biological sample from a subject for whom one or more autoimmune disorders to be handled, through the sample collection module 108;
extracting one or more DNA sequences from the biological sample, using one or more DNA sequence extraction techniques, through the DNA extraction and sequencing module 110;
identifying microbial taxa from the one or more DNA sequences of the biological sample, using one or more microbial taxa extraction techniques, wherein the microbial taxa is composed of one or more microbes predominantly present in the biological sample, through the DNA extraction and sequencing module 110;
identifying one or more pathogenic microbes, by mapping pathogenic microbial taxa obtained from the microbial taxa to the pathogenic microbial taxa present in a disease microbe map (DM map) of the epitope knowledgebase 116;
identifying one or more microbial peptides from the biological sample, using one or more microbial peptides identification techniques, through the peptide identification module 112;
identifying, one or more microbial epitopes, from the one or more microbial peptides present in the biological sample, using the epitope knowledgebase 116, through the epitope identification module 114;
assessing the one or more autoimmune disorders of the subject, based on at least one of (i) the one or more pathogenic microbes and (ii) the one or more microbial epitopes, via one or more hardware processors, using the epitope knowledgebase 116, through the disease assessment module 118; and
generating an artificial sequence construct, using one or more mimic epitopes identified from the epitope knowledgebase 116 through the sequence construct generation module 120, wherein the artificial sequence construct comprises of one or more refined molecular mimic epitopes associated to the one or more autoimmune disorders present in the epitope knowledgebase 116, linked by (i) one or more peptide linkers, (ii) one or more adjuvants, and (iii) one or more toll-like receptor (TLR) ligands, to enhance an immunogenicity.

9. The system as claimed in claim 8, wherein
the one or more refined molecular mimic epitopes associated to the one or more autoimmune disorders are utilized to generate one or more monoclonal antibodies for administering the subject to impede a disease progression;
the one or more peptide linkers are selected from a group consisting of: GGGS, AAY, KK, GPGPG, and HEYGAEALERAG, and wherein B cell epitopes and HTL epitopes (MHC II epitopes) can be linked by KK and GPGPG peptide linker;
the one or more adjuvants are selected from a group consisting of: particulate emulsions, microparticles, iscoms cochleates, and liposomes, and wherein the one or more adjuvants are fused using a EAAAK linker;
the one or more toll-like receptor (TLR) ligands are selected from a group consisting of: MPL (TLR2 and TLR4 ligands), CpG ODN (TLR9 ligand), CTB-CpG, Flagellin (TLR5 ligand); and
the one or more pathogenic microbes comprising the epitope identified in a disease condition is eliminated using antimicrobials, wherein the antimicrobials comprise a microbial modulation in the form of probiotics, prebiotics or the antimicrobials that target the one or more pathogenic microbes in the biological sample in order to control disease progression.

10. The system as claimed in claim 8, wherein
the artificial sequence construct comprises a combination of one or more epitope sequences from a sequence identifier (ID) D2_1 to D2_16 listed in Table 4, the one or more peptide linkers, the one or more adjuvants or the one or more toll-like receptor (TLR) ligands, for an autoimmune disorder being a primary biliary cholangitis (PBC); and
the artificial sequence construct comprises a combination of one or more epitope sequences from a sequence identifier (ID) D1_1 to D1_35 listed in Table 5, the one or more peptide linkers, the one or more adjuvants or the one or more toll-like receptor (TLR) ligands, for an autoimmune disorder being an Atopic Dermatitis (AD).

11. The system as claimed in claim 8, wherein the one or more hardware processors 102 are configured to perform assessing an efficacy of the artificial sequence construct generated to the subject, through the efficacy assessment module 122.

12. The system as claimed in claim 8, wherein the epitope knowledgebase 116 comprises a DM_map, a X_RMME_map, and a X_RMME_detail_map, wherein the DM_map comprises mapping of the one or more autoimmune disorders to a set of microbes, the X_RMME_map comprises mapping of one or more refined molecular mimic epitopes to each of the one or more autoimmune disorders, and the X_RMME_detail_map comprises a detailed information of each of the one or more refined molecular mimic epitopes present in the X_RMME_map.

13. The system as claimed in claim 8, wherein
the one or more refined molecular mimic epitopes of an autoimmune disorder being a primary biliary cholangitis (PBC), are listed in Table 4 in the form of epitope sequences from a sequence identifier (ID) D2_1 to D2_16; and
the one or more refined molecular mimic epitopes of an autoimmune disorder being an Atopic Dermatitis (AD), are listed in Table 5 in the form of epitope sequences from a sequence identifier (ID) D1_1 to D1_35.

14. The system as claimed in claim 8, wherein the one or more hardware processors 102 are configured to create the epitope knowledgebase 116, by:
identifying a plurality of disease specific proteomes pertaining to the one or more autoimmune disorders, using one or more data mining techniques;
predicting one or more disease specific epitopes for each of the one or more autoimmune disorders, from the plurality of disease specific proteomes, based on a binding capability;
identifying one or more potential molecular mimic epitopes, for each of the one or more autoimmune disorders, from the one or more disease specific epitopes, that show a sequence similarity with self-peptides and results in cross-activation of autoreactive T; and
refining the one or more potential molecular mimic epitopes, to obtain one or more refined molecular mimic epitopes for each of the one or more autoimmune disorders, using one or more of (i) a structural superimposition and analysis, (ii) a cellular localization prediction, (iii) a proteosome processing, and (iv) an immunogenicity prediction.

15. One or more non-transitory machine-readable information storage mediums comprising one or more instructions which when executed by one or more hardware processors cause:
collecting a biological sample from a subject for whom one or more autoimmune disorders to be handled;
extracting one or more DNA sequences, from the biological sample, using one or more DNA sequence extraction techniques;
identifying microbial taxa from the one or more DNA sequences of the biological sample, using one or more microbial taxa extraction techniques, wherein the microbial taxa is composed of one or more microbes predominantly present in the biological sample;
identifying one or more pathogenic microbes, by mapping pathogenic microbial taxa obtained from the microbial taxa to the pathogenic microbial taxa present in a disease microbe map (DM map) of an epitope knowledgebase;
identifying one or more microbial peptides from the biological sample, using one or more microbial peptides identification techniques;
identifying one or more microbial epitopes, from the one or more microbial peptides present in the biological sample, using the epitope knowledgebase;
assessing one or more autoimmune disorders of the subject, based on at least one of (i) the one or more pathogenic microbes and (ii) the one or more microbial epitopes, using the epitope knowledgebase;
generating an artificial sequence construct, using one or more mimic epitopes identified from the epitope knowledgebase, wherein the artificial sequence construct comprises of one or more refined molecular mimic epitopes associated to the one or more autoimmune disorders present in the epitope knowledgebase linked by (i) one or more peptide linkers, (ii) one or more adjuvants, and (iii) one or more toll-like receptor (TLR) ligands, to enhance an immunogenicity; and
assessing an efficacy of the artificial sequence construct generated to the subject.
